# EUROPEAN PATENT SPECIFICATION

(11) **EP 3 244 909 B1**
(45) Date of publication and mention of the grant of the patent: **09.10.2019**
(21) Application number: 16700619.6
(22) Date of filing: 14.01.2016
(51) Int. Cl.: A61K 38/06, A61K 8/64, A61P 43/00

(54) **PROTEASOME INHIBITORS FOR TREATING A DISORDER RELATED TO AN ACCUMULATION OF NON-DEGRADED ABNORMAL PROTEIN OR A CANCER**
PROTEASOMINHIBITOREN ZUR BEHANDLUNG VON VORZEITIGEN ALTERUNGSTÖRUNGEN
INHIBITEURS DU PROTÉASOME POUR TRAITER DES TROUBLES DE VIEILLISSEMENT PRÉMATURÉ

(30) Priority: 14.01.2015 EP 15305026
(43) Date of publication of application: 22.11.2017
(73) Proprietor: Université d'Aix-Marseille, 13007 Marseille (FR); Assistance Publique Hôpitaux de Marseille, 13005 Marseille (FR); INSERM (Institut National de la Santé et de la Recherche Médicale), 75013 Paris (FR); Association Francaise Contre Les Myopathies, 75013 Paris (FR); CENTRE NATIONAL DE LA RECHERCHE SCIENTIFIQUE - CNRS, 75016 Paris 16 (FR); Progelife, 13001 Marseille (FR)
(72) Inventor: LEVY, Nicolas, 13008 Marseille (FR); CAU, Pierre, 13540 Puyricard (FR); DE SANDRE-GIOVANNOLI, Annachiara, 13400 Aubagne (FR); HARHOURI, Karim, 13700 Marignane (FR); PERRIN, Sophie, 13009 Marseille (FR); NAVARRO, Claire, 13005 Marseille (FR); CHARTIER, Aymeric, 34000 Montpellier (FR); SIMONELIG, Martine, 34830 Clapiers (FR)
(74) Representative: Cabinet Plasseraud
(86) International application number: PCT/EP2016/050678
(87) International publication number: WO 2016/113357

(56) References cited:
- WO-A1-2008/148016
- WO-A1-2011/076880
- WO-A2-2008/029414
- FR-A1- 2 926 020
- US-A1- 2014 161 785
- E. T. EFUET: "Farnesyl and Geranylgeranyl Transferase Inhibitors Induce G1 Arrest by Targeting the Proteasome", CANCER RESEARCH, vol. 66, no. 2, 15 January 2006 (2006-01-15), pages 1040-1051, XP055188211, ISSN: 0008-5472, DOI: 10.1158/0008-5472.CAN-05-3416
- ISHITA S. MEHTA ET AL: "Progeria, the nucleolus and farnesyltransferase inhibitors", BIOCHEMICAL SOCIETY TRANSACTIONS, vol. 38, no. 1, 1 February 2010 (2010-02-01), page 287, XP055188231, ISSN: 0300-5127, DOI: 10.1042/BST0380287
- GONZALEZ JOSE M ET AL: "A-type lamins and Hutchinson-Gilford progeria syndrome: pathogenesis and therapy", FRONTIERS IN BIOSCIENCE (SCHOLAR EDITION), UNITED STATES, vol. 3S, no. 3, 1 January 2011 (2011-01-01), pages 1133-1146, XP008160120, ISSN: 1945-0524, DOI: 10.2741/216 [retrieved on 2001-06-01]
- RIVERA-TORRES JOSÉ ET AL: "Identification of mitochondrial dysfunction in Hutchinson-Gilford progeria syndrome through use of stable isotope labeling with amino acids in cell cul", JOURNAL OF PROTEOMICS, vol. 91, 2013, pages 466-477, XP028766416, ISSN: 1874-3919, DOI: 10.1016/J.JPROT.2013.08.008
- PIERRE CAU ET AL: "Nuclear matrix, nuclear envelope and premature aging syndromes in a translational research perspective", SEMINARS IN CELL & DEVELOPMENTAL BIOLOGY, vol. 29, 1 May 2014 (2014-05-01), pages 125-147, XP055188279, ISSN: 1084-9521, DOI: 10.1016/j.semcdb.2014.03.021
- VEENA K PARNAIK ET AL: "Lamins, laminopathies and disease mechanisms: Possible role for proteasomal degradation of key regulatory proteins", JOURNAL OF BIOSCIENCES, SPRINGER-VERLAG, INDIA, vol. 36, no. 3, 23 July 2011 (2011-07-23), pages 471-479, XP019946469, ISSN: 0973-7138, DOI: 10.1007/S12038-011-9085-2
- NA GUO ET AL: "MG132, a proteasome inhibitor, induces apoptosis in tumor cells", ASIA-PACIFIC JOURNAL OF CLINICAL ONCOLOGY, vol. 9, no. 1, 15 May 2012 (2012-05-15), pages 6-11, XP55252390, ISSN: 1743-7555, DOI: 10.1111/j.1743-7563.2012.01535.x

## Description

### FIELD

The disclosure relates to the use of proteasome inhibitors in the treatment and/or prevention of a disorder related to an accumulation of a non-degraded abnormal protein, such as progerin and/or farnesylated prelamin A, and particularly a premature ageing disorder linked to these above abnormal proteins.

The disclosure also relates to the dermatological, dermocosmetic or cosmetic use of a proteasome inhibitor for preventing and/or attenuating progerin- and/or farnesylated prelamin A-linked skin ageing. The disclosure also relates to the use of a proteasome inhibitor for attenuating progerin- and/or farnesylated prelamin A-linked physiological ageing.

The disclosure also relates to the use of proteasome inhibitors in the treatment and/or prevention of age-related disorders and their metabolic consequences associated with the presence and/or accumulation of progerin and/or farnesylated prelamin A, in particular in tissues including skin, adipose tissue, bone, blood vessels including endothelial and vascular smooth muscle cells and their pathological consequences on skin, heart, brain or kidney.

The disclosure also relates to the use of proteasome inhibitors in the treatment of disorders associated with the presence and/or accumulation of toxic protein during genetic neurodegenerative and/or muscle disease.

The disclosure also relates to the use of proteasome inhibitors in the treatment of disorders associated with the overexpression of SRSF1, such as cancer.

### BACKGROUND

Hutchinson-Gilford progeria syndrome (HGPS; OMIM #176670) is a rare genetic disorder which affects 1 in 4-8 million children with symptoms resembling normal adult ageing that include growth impairment, very thin skin, loss of subcutaneous fat, alopecia, osteoporosis and heart disease leading to shortened life span and death at about 13.5 years (*1*, *2*). This syndrome is caused by a *de novo* missense point mutation c.1824 C>T, p.G608G within exon 11 of the *LMNA* gene that encodes lamin A (*3, 4*). This mutation activates a cryptic donor splice site in exon 11 that leads to deletion of 50 amino acids at the carboxy-terminal globular domain resulting in a truncated and permanently farnesylated prelamin A called progerin. This protein is lacking residues 607-656 of prelamin A but retaining the C-terminal CAAX box, a target for farnesylation. Because an endoproteolytic cleavage site is lost in the truncated lamin/progerin, it remains permanently farnesylated. Lamins A/C, together with the B-type lamins, are the major components of the nuclear lamina, a fibrous network underlying the inner nuclear membrane.

At the cellular level, HGPS premature ageing disorder is characterized by dramatic defects in nuclear envelope structure, large-scale alterations in nuclear shape, blebbing, "herniations", loss of some inner nuclear membrane (INM) proteins from one pole of the nucleus and disruption of the underlying heterochromatin. Because lamin A is also a component of the internal nuclear matrix, its alteration in HGPS patient cells might affect the distribution and/or the structural organization of nuclear functional areas such as nucleoli, speckles and nuclear bodies. Abnormalities in nuclear matrix composition also result in defects in DNA and RNA metabolism steps, from DNA repair, leading to genome instability, to RNA transcription and splicing. Nuclear metabolic defects as well as their consequences on cell cycle, metabolic pathways and cell compartment functions lead to cellular senescence (*5*).

Progerin has been shown to be produced without mutation in *LMNA* gene during physiological ageing (*6*) and is considered as a biomarker of ageing in man (*7*) and mice skin, where progerin synthesis has been shown to be triggered by UV-induced ROS production (*8*) mainly by NADPH oxidase NOX1 overactivity (*9*). Both phenomena have been observed in a DNA repair genetic disease, *Xeroderma pigmentosum* type C (XPC), both in human cultured keratinocytes from patients and in *Xpc*^{*-*/*-*} mice (*9*). A comparable overactivity of NOX1 has been reported in other DNA repair diseases and could also lead to progerin production (*10*). Telomere shortening during replicative senescence in cultured cells induced progerin synthesis (*11*). Cells from mesenchymal lineage, such as endothelial and blood vessel smooth muscle cells, bone and adipose tissue cells, skeletal and heart muscle cells are more specifically sensitive to progerin, which induces the progressive disappearance of adult stem cells responsible of renewal of these tissues (*12*), a phenomenon also induced by farnesylated prelamin A and observed in adult epidermal stem cells (*13*). On the other hand, oxidative stress resulted in the blockade of ZMPSTE24 both at mRNA and protein levels resulting in the production of farnesylated prelamin A during aging of smooth muscle cells from blood vessels (*14*). Between the ages of 1 month and 97 years, progerin staining increased in coronary arteries from non HGPS subjects (*15*). Furthermore, during endothelia senescence, the mislocalisation of SRSF1 from nucleus to cytosol resulted in abnormal splicing events in several mRNAs, among them prelamin A mRNA (*16*). Neither progerin nor farnesylated prelamin A were produced in brain neurons and astrocytes, due to the miR-9 induced cleavage of prelamin A mRNA (*17, 18*). However, progerin or farnesylated prelamin A could indirectly contribute to brain ageing through their synthesis by vascular (*19*) and ependymal cells (*20*) in response to oxidative stress (*21, 22*) together with lamin B accumulation in neural cells (*23*).

The strategies for treating HGPS, as well as other progeroid syndromes linked to prelamin A processing, can be divided into three main axes: i/ decreasing the toxicity of farnesylated prelamin A, notably through the blockade of the isoprenoid biosynthesis or through the blockade of its synthesis; ii/ blocking the *LMNA* exon 11 cryptic splicing site leading to mature progerin mRNA then to progerin synthesis; i i i / degrading progerin or farnesylated prelamin A within cells.

Only the first strategy led to therapeutic trials in human patients up to date (*5*).

A farnesyl transferase inhibitor (FTI), lonafarnib, has been tested (*24*) but its benefits are controversial (*25*). Since RAS is well established as the most frequently mutated oncogene in human cancer and both normal and oncogenic RAS require farnesylation, Farnesyltransferase (Ftase) has been one of the most attractive targets for anti-cancer drug discovery. A large number of Ftase inhibitors (FTIs) have been developed and tested in more than 70 clinical trials (*26*). The results were largely disappointing, however, with the surprising finding that H-RAS and K-RAS can still be geranylgeranyled when Ftase is inhibited (*27*). Moreover, one FTI, FTI-277, has been shown to inhibit proteasome activity (*28*). However, none of the FTI previously used in cultured cells from progeria patients, in several mice models where FTI efficacy was far lower than in cultured cells (*29*) and in progeria clinical trials (*24*), have evidenced an inhibitory activity towards proteasome or have shown a decrease in the amount of progerin or of prelamin A as described in the present disclosure.

Another treatment, which was tested between 2008 and 2013, is the combination of zoledronate (an aminobisphosphonate inhibitor of farnesyl-pyrophosphate synthase, Zo) and pravastatin (statin inhibitor of HMG-CoA reductase, Pra), i.e. ZoPra. A third clinical trial combining ZoPra with lonafarnib is in progress.

The second strategy has been validated both in cultured HGPS cells as well as in a mouse model of progeria. Short oligonucleotides bind to *LMNA* exon 11 cryptic splicing site activated by *LMNA* mutation and lead to the reduction of progerin mRNA and protein biosynthesis in HGPS cells (*30*). The intravenous administration of two morpholino-oligonucleotides rescue the life span as well as several biological parameters of the KI mouse model G609G, reproducing both the pathophysiological mechanism, clinical and biological symptoms of progeria (*31*). One morpholino targets the *LMNA* exon 11 cryptic splicing site and inhibits progerin mRNA production, whereas the second targets *LMNA* exon 10 splicing site, thus favoring the production of lamin C mRNA and lowering the production of lamin A mRNA. In the same progeria KI mouse model, it has been demonstrated that the splicing factor SRSF1 binds to *LMNA* exon 11 cryptic site and favors the production of progerin mRNA (*32*).

The third strategy, the degradation of progerin or of farnesylated prelamin A within cells, has been studied. The immunosuppressor rapamycin, through activation of macroautophagy, lowers progerin in HGPS fibroblasts and rescues their nuclear shape phenotype (*33, 34*) and subsequent Erratum 2013). Thus, rapamycin has been proposed to constitute a suitable treatment of HGPS. Lamin B1 anchored through a farnesyl group to nuclear lamina has been shown to be exported to cytosol and degraded through autophagy in cultured cells upon oncogenic insults, such as by activated RAS (*35*).

However, there is still a need for an efficient treatment of HGPS and other progeroid syndromes having a related, similar or identical pathophysiological mechanism. Said mechanism may involve the presence of truncated and/or farnesylated prelamin A and/or B-type lamins, and/or duplication of *LMNA, LMNB1* and/or *LMNB2.* Such a treatment could also be beneficial to prevent and/or to treat diseases accompanying lamin-linked physiological ageing.

### SUMMARY

The invention is defined in the claims.

As shown in the Example 1, the inventors have surprisingly discovered that the use of a proteasome inhibitor, such as MG132, induces progerin clearance in HGPS cells. Indeed, MG132 has two independent functions in HGPS cells:
- on one hand, it induces autophagy and thus protein degradation, and
- on the other hand, it reduces the amount of the transcription factor called SRSF1, which is involved in prelamin A mRNA splicing leading to progerin mRNA (*32*).

As a way of consequence, progerin is both degraded by autophagy and less produced because of the downregulation of SRSF1 .

The inventors have also surprisingly shown that the use of a proteasome inhibitor is efficient for treating cancer cells (see Example 2) and for treating an animal model of OculoPharyngeal Muscular Dystrophy (OPMD) (see Example 3).

The present disclosure thus relates to a proteasome inhibitor for use for treating and/or preventing a disorder related to an accumulation of a non-degraded abnormal protein, such as a truncated and/or farnesylated prelamin A, particularly leading to a premature ageing disorder.

Preferably, the proteasome inhibitor is for use for treating and/or preventing a disorder related to an accumulation of a non-degraded abnormal protein, such as truncated and/or farnesylated prelamin A leading to a premature ageing disorder, by downregulating SRSF1.

The present disclosure also relates to a method for treating and/or preventing a disorder related to an accumulation of a non-degraded abnormal protein, such as truncated and/or farnesylated prelamin A leading to a premature ageing disorder, in a subject in need thereof, comprising administering to said subject a therapeutically effective amount of a proteasome inhibitor.

The present disclosure also relates to the dermatologic, dermocosmetic or cosmetic use of a proteasome inhibitor for preventing and/or attenuating skin ageing, particularly human skin ageing. The disclosure also relates to the use of a proteasome inhibitor for attenuating physiological ageing, such as progerin- and/or farnesylated prelamin A-linked physiological ageing.

The disclosure also relates to the use of proteasome inhibitors in the treatment and/or prevention of age-related disorders and their metabolic consequences, in particular those including tissues expressing progerin and/or farnesylated prelamin A, such as skin, adipose tissue, bone, blood vessels and their endothelial and vascular smooth muscle cells and their consequences on skin, heart, brain or kidney.

### DETAILED DESCRIPTION

### Definitions

As used herein, the term "proteasome inhibitor" is a compound which blocks the enzymatic activity of proteasome. Mammalian 26S cytosolic and nuclear proteasome is made of one 20S proteolytic core and two 19S regulatory cap subunits (*36*). The 20S core provides a barrel-shaped cavity in which proteins are degraded.

The 20S core is made of four rings, two α and two β. Each α-ring comprises seven distinct α-subunit encoded by 7 genes, and each β-ring comprises seven distinct β- subunits encoded by 7 genes.

The 19S regulatory cap subunit is composed of a base comprising 6 ATPases and 2 non-ATPase subunits, and a lid containing up to 10 non-ATPase subunits.

As used herein, the terms "treating" or "treatment" means reversing, alleviating, inhibiting the progress of, or preventing the disorder or condition to which such term applies, or one or more symptoms of such disorder or condition.

As used herein, the term "preventing" or "prevention" refers to alleviating the disease or condition from occurring in a subject which has not yet been diagnosed as having it.

A "therapeutically effective amount" is intended for a minimal amount of active agent which is necessary to impart therapeutic benefit to a subject. For example, a "therapeutically effective amount" is an amount which induces, ameliorates or otherwise causes an improvement in the pathological symptoms, disease progression or physiological conditions associated with a disease or which improves resistance to a disorder.

As used herein, the term "subject" denotes a mammal, such as a rodent, a feline, a canine, and a primate. Preferably a subject according to the disclosure is a human. All the treatments and methods of the disclosure may apply to mammals, particularly non-human mammals, as well as humans. Preferably, they may apply to humans.

### Treatment of disorders related to an accumulation of a non-degraded abnormal protein according to the disclosure

The present disclosure relates to a proteasome inhibitor for use for treating and/or preventing a disorder related to an accumulation of a non-degraded abnormal protein, such as truncated and/or farnesylated prelamin A and/or B-type lamins, or the proteins produced by a duplication of *LMNB1* and/or *LMNB2.*

As used herein, the "disorder related to an accumulation of a non-degraded abnormal protein" is a disorder which involves the presence of an abnormal protein, said protein being accumulated in the cell because non degraded. Said abnormal protein may be a misfolded protein, or a mutant protein. The accumulation of non-degraded abnormal protein may be nuclear and/or cytosolic. Preferably, the non-degraded abnormal protein is a truncated and/or farnesylated prelamin A, a truncated and/or farnesylated B-type lamin, or a protein produced by a duplication of *LMNB1* and/or *LMNB2.* A preferred example of a truncated and farnesylated prelamin A is progerin.

Preferably, the disorder related to an accumulation of a non-degraded abnormal protein is a disorder related to a nuclear accumulation of a non-degraded abnormal protein. Preferably, said disorder is chosen from premature ageing disorders, striated muscle and neurodegenerative disorders. In said disorders, there is accumulation of a non-degraded abnormal protein in the nucleus.

Preferably, the disorder related to an accumulation of a non-degraded abnormal protein is a disorder related to a cytosolic accumulation of a non-degraded abnormal protein.

Preferably, said disorder is chosen from striated muscle and neurodegenerative disorders. Among the striated muscle and neurodegenerative disorders, one can preferably quote OculoPharyngeal Muscular Dystrophy (OPMD). The use of proteasome inhibitors on an OPMD model is shown in Example 3.

More preferably, according to the disclosure, said disorder is chosen from premature ageing disorders. Premature ageing disorders are characterized by an accelerated ageing, either affecting multiple or all tissues and causing affected individuals to exhibit only some of the features associated with ageing (which is called "segmental"), or affecting only one tissue (which is called "unimodal"). For example, premature ageing disorders may be chosen from disorders involving an accelerated ageing, and in which at least one of the following mutations is present:
- prelamin A and/or lamins B are truncated, and/or
- progerin and/or prelamin A and/or lamins B are farnesylated, and/or
- *LMNA, LMNB1* and/or *LMNB2* are duplicated.

For example, if *LMNB1* is duplicated, then this genetic defect leads to adult-onset autosomal dominant leukodystrophy (*37*).

Premature ageing disorders may be chosen among progeroid syndromes.

Progeroid syndromes are heritable human disorders including clinical symptoms that remind ageing but appearing prematurely and whose evolution is dramatically accelerated. Most human syndromes of accelerated ageing are caused by one of two major mechanisms: defects in the nuclear lamina and matrix proteins or alterations in proteins involved in DNA repair (*5*).

Among the progeroid syndromes, one can quote Hutchinson- Gilford progeria syndrome (HGPS), atypical progeria syndrome (APS), restrictive dermopathy, Nestor-Guillermo progeria syndrome, due to mutation in *LMNA, ZMPSTE24* or *BANF1* respectively, and Werner, Bloom, Rothmund-Thomson, or Cockayne syndromes, *Xeroderma pigmentosum* and trichothiodystrophy, due to mutations in genes whose products are involved in DNA repair.

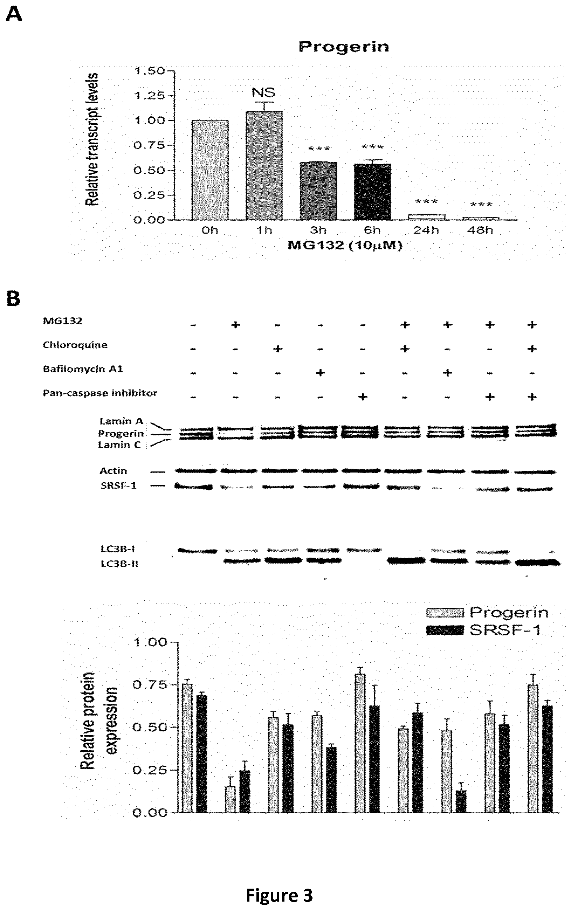

Preferably, the proteasome inhibitor is for use for treating and/or preventing a disorder related to an accumulation of a non-degraded abnormal protein by downregulating SRSF1. SRSF1 is a serine/arginine-rich splicing factor 1, which belongs to the serine/arginine-rich splicing factor family. By "downregulating SRSF1", it is meant that the proteasome inhibitor downregulates the SRSF1 protein by at least 10%, preferably by at least 50%, preferably by at least 60%, as compared to an untreated control. The downregulation of SRSF1 may be measured as described in the Example 1 and Figure 3B. Without being bound by a theory, the proteasome inhibitor for use according to the present disclosure may exert its action by inducing a caspase-mediated reduction of SRSF1-levels, which leads to SRSF1 downregulation. This downregulation then decreases progerin transcript levels, and thus decreases progerin production.

Preferably, the proteasome inhibitor which is used for treating and/or preventing a disorder related to an accumulation of a non-degraded abnormal protein according to the disclosure is a chemical compound. Preferably, it does not comprise MG132. More preferably, it is chosen from peptides, optionally modified. More preferably, it is chosen from peptides, optionally modified, except MG132.

The peptides are preferably tripeptides, optionally modified. Preferably, these peptides are tripeptides comprising at least two leucine, optionally modified. The modification may be addition of an aldehyde function, or addition of a chemical compound, like -B(OH)2.

Preferably, these peptides include Z-Leu-Leu-Leu-B(OH)2, Z-Leu-Leu-Nva-H, Z-Leu-Leu-Phe-al and Z-Leu-Leu-Leu-al.

The modified tripeptide Z-Leu-Leu-Leu-B(OH)2 is also known under the name MG262.

The modified tripeptide Z-Leu-Leu-Nva-H is also known under the name MG115.

The modified tripeptide Z-Leu-Leu-Leu-al is also known under the name MG132. The modified tripeptide Z-Leu-Leu-Phe-al is also known under the name MG110.

More preferably, the proteasome inhibitor is chosen from MG115, MG262, MG110 and MG132. More preferably, the proteasome inhibitor is chosen from MG115, MG110 and MG262.

The disclosure also relates to pharmaceutical compositions comprising a proteasome inhibitor of the disclosure, particularly chosen from MG115, MG262, MG110 and MG132.

When the proteasome inhibitor of the disclosure is a peptide, it may be conjugated or attached to an agent which increases the accumulation of said peptide in a cell.

Such conjugation with a suitable agent would render said peptide more membrane permeable, such as cell membrane permeable carriers. Such an agent may be a cell membrane permeable carrier, for example a positively charged amino acid-rich peptide, preferably an arginine-rich peptide. Preferably, arginine-rich peptide is a polyarginine tag having the sequence RRRRRRRRR (SEQ ID NO:12). Other cell penetrating peptides may be chosen among the NGR peptide derived from the aminopeptidase (CD13) N Ligand (CNGRCG), Antennapedia Leader Peptide (KKWKMRRNQFWVKVQRG SEQ ID NO:13), Bcl-2 Binding Peptide (Decanoyl-KNLWAAQRYGRELRRMSDEFEGSFKGL SEQ ID NO:14), a tat sequence (RKKRRQRRR SEQ ID NO:15), buforin (TRSSRAGLQFPVGRVHRLLRK SEQ ID NO:16), a peptidic fragment of the Human T-cell Lymphotrophic Virus (HTLV)-11 Rex (TRRQRTRRARRNR SEQ ID NO:17), the lipid membrane translocating peptide (KKAAAVLLPVLLAAP SEQ ID NO:18) and penetratin (RQIKIWFQNRRMKWKKGG SEQ ID NO:19).

When the proteasome inhibitor of the disclosure is a peptide, it may be administered in form of one of its pharmaceutically active salts. Suitable pharmaceutically active salts comprise acid addition salts and alkali or earth alkali salts. For instance, sodium, potassium, lithium, magnesium or calcium salts can be obtained. The peptide forms pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, mandelic acid, o-methylmandelic acid, hydrogen-benzenesulfonic acid, picric acid, adipic acid, D-o-tolyl tartaric acid, tartronic acid, a-toluic acid, (o, rn, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

The peptide of the present disclosure may also be conjugated to a non-peptide moiety. A polymer molecule to be coupled to the peptide may be any suitable polymer molecule, such as a natural or synthetic homopolymer or heteropolymer, typically with a molecular weight in the range of about 300-100,000 Da, such as about 500-20,000 Da. Examples of suitable polymer molecules include polymer molecules selected from the group consisting of polyalkylene oxide (PAO), including polyalkylene glycol (PAG), such as polyethylene glycol (PEG) and polypropylene glycol (PPG), branched PEGs, poly-vinyl alcohol (PVA), poly-carboxylate, poly-(vinylpyrrolidone), polyethylene-co-maleic acid anhydride, polystyrene-co-maleic acid anhydride, dextran, including carboxymethyldextran, or any other biopolymer suitable for reducing immunogenicity and/or increasing functional in vivo half-life and/or serum half-life. Another example of a polymer molecule is human albumin or another abundant plasma protein. Generally, polyalkylene glycol-derived polymers are biocompatible, non-toxic, non-antigenic, non-immunogenic, have various water solubility properties, and are easily excreted from living organisms.

Therefore, a proteasome inhibitor of the disclosure may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the disclosure can be formulated for a topical, oral, parenteral, intranasal, intragastral, intravenous, intramuscular, subcutaneous, intraperitoneal, intravaginal, rectal, sublingual, transdermal or intraocular administration and the like.

Administration forms include, for example, pills, tablets, film tablets, coated tablets, capsules, liposomal formulations, micro- and nano-formulations, powders and deposits.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment. To prepare pharmaceutical compositions, an effective amount of the proteasome inhibitor may be dissolved or dispersed in a pharmaceutically acceptable carrier or aqueous medium. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The carrier can also be a solvent or dispersion medium containing, for example, water, ethanol, polyol (for example, glycerol, propylene glycol, and liquid polyethylene glycol, and the like), suitable mixtures thereof, and vegetal oils.

The proper fluidity can be maintained, for example, by the use of a coating, such as lecithin, by the maintenance of the required particle size in the case of dispersion and by the use of surfactants.

The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization.

Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose.

These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

In addition to the compounds formulated for parenteral administration, such as intravenous or intramuscular injection, other pharmaceutically acceptable forms include, e.g. tablets or other solids for oral administration; time release capsules; and any other form currently used.

The proteasome inhibitor may be used alone, or in combination with at least one other drug.

Such drug may be chosen from anti-inflammatory agents (*38*); antisense oligonucleotides, like 5'-GCTACCACTCACGTGGTGGTGATGG-3' (SEQ ID NO:1) and 5'- GGGTCCACCCACCTGGGCTCCTGAG-3' (SEQ ID NO:2), which are known from (*31*); aminobisphosphonates inhibitors of farnesyl-pyrophosphate synthase, like zoledronate; statins inhibitors of HMG-CoA reductase, like pravastatin; other peptides such as NOX1 inhibitors; activators of chaperone or of their ATPase activity; autophagy activators such as trehalose, lithium salts, spermidine; Rho kinase inhibitors, such as fasudil hydrochloride, Y-27632, HA1077 or H89; Rho inhibitors, such as BA-210 (Cethrin®) of BioAxone Therapeutics; and mixtures thereof.

Such drug may also be chemotherapy or immunotherapy.

The present disclosure also relates to the dermatological, dermocosmetic or cosmetic use of a proteasome inhibitor for preventing and/or attenuating skin ageing, particularly human skin ageing. The present disclosure also relates to the use of a proteasome inhibitor for attenuating physiological ageing.

The proteasome inhibitor is as described above.

It may be used for preventing and/or attenuating skin ageing, and particularly for reducing wrinkles, fine lines, and/or for preventing thinning of the skin and/or an aspect of soft and withered skin.

The disclosure also relates to proteasome inhibitors for use in the treatment and/or prevention of age-related disorders and their metabolic consequences, in particular those including tissues expressing progerin and/or farnesylated prelamin A, such as skin, adipose tissue, bone, blood vessels and their endothelial and vascular smooth muscle cells and their consequences on skin, heart, brain or kidney. Particularly, the age-related disorder may be chosen from osteoporosis, diabetes of type 2 and arteriosclerosis. Thus, preferably, the disclosure relates to a proteasome inhibitor for use for treating and/or preventing age-related disorders and their metabolic consequences, in particular those including tissues expressing progerin and/or farnesylated prelamin A, such as skin, adipose tissue, bone, blood vessels and their endothelial and vascular smooth muscle cells and their consequences on skin, heart, brain or kidney. Preferably, said age-related disorder is chosen from osteoporosis, diabetes of type 2 and arteriosclerosis.

Administration of the proteasome inhibitor may be by any suitable method as described above. Suitable amounts of the proteasome inhibitor may be determined empirically, but typically are in the range given below. A single administration of proteasome inhibitor may be sufficient to have a beneficial effect for the patient, but it will be appreciated that it may be beneficial if it is administered more than once, in which case typical administration regimes may be, for example, once or twice a week for 2-4 weeks every six months, or once a day for a week every four to six months.

Dosages for administration will depend upon a number of factors including the nature of the composition, the route of administration and the schedule and timing of the administration regime.

Suitable doses of a molecule or a combination of molecules of the disclosure may be in the order of 1 µg up to 10 µg, up to 15 µg, up to 20 µg, up to 25 µg, up to 30 µg, up to 35 µg, up to 50 µg, up to 100 µg, up to 500 µg or more per administration. Suitable doses may be less than 15 µg, but at least 1 ng, or at least 2 ng, or at least 5 ng, or at least 50 ng, or least 100 ng, or at least 500 ng, or at least 1 µg, or at least 10 µg. For some molecules of the disclosure, the dose used may be higher, for example, up to 1 mg, up to 2 mg, up to 3 mg, up to 4 mg, up to 5 mg or higher. Such doses may be provided in a liquid formulation, at a concentration suitable to allow an appropriate volume for administration by the selected route.

### Diagnosis method according to the disclosure

The disclosure also relates to a method for diagnosing a disorder related to an accumulation of a non-degraded abnormal protein, particularly a premature ageing disorder, in a biological sample of a subject, comprising i) the detection of thread-like PML-NBs in cell nucleus, and ii) the detection of a secondary parameter. Said secondary parameter may be chosen from a clinical symptom of ageing of the subject, and/or the detection of DNA breakage in the biological sample.

Indeed, as shown in the example, the inventors have discovered that thread-like PML-NBs are markers of HGPS cells, and thus may be usable in diagnostic methods of a disorder related to an accumulation of a non-degraded abnormal protein, particularly a premature ageing disorder.

PML-NBs (ProMyelocytic Leukemia-Nuclear Bodies) are nuclear bodies and have been described as discrete nuclear speckles tightly associated with the nuclear matrix (*39*).

The PML gene was initially identified as the fusion partner of the retinoic acid receptor α gene in the T(15;17) chromosomal translocation in acute promyelocytic leukemia. PML-NBs range in size from 0.2 µm to 1.2 µm in diameter and they are dynamic structures that change size, position and number in response to stress such as heat shock, heavy metal exposure and DNA damage.

PML-NBs may be detected, thanks to classical methods known in the art, like immunofluorescence, Western blot or flow cytometry.

Thus, the method for diagnosing a disorder related to an accumulation of a non- degraded abnormal protein, particularly a premature ageing disorder (more particularly HGPS and other progeroid syndromes), in a biological sample of a subject, according to the disclosure, comprises i) detecting thread-like PML-NBs in said biological sample, and ii) detecting of a secondary parameter. If thread-like PML-NBs are detected, and if the secondary parameter is detected, then it can be concluded that the subject is afflicted by a disorder related to an accumulation of a non-degraded abnormal protein.

The biological sample may be cells from a skin biopsy, peripheral blood cells, epithelial cells from jugal mucosa or urine.

### Cancer treatment according to the disclosure

Finally, the disclosure also relates to a proteasome inhibitor as described above, and which downregulates SRSF1, for treating and/or preventing cancers. Said application is surprisingly shown in Example 2 below.

Said proteasome inhibitor may be as described above. Preferably, said proteasome inhibitor may be chosen from MG132, MG115, MG262 and MG110.

Overexpression of SRSF1 proto-oncogene in human cancers has been observed (*40*).

Thus, by downregulating SRSF1, the proteasome inhibitors of the disclosure are able to treat cancers.

The cancer may be of any type. Preferably, the cancer is chosen from breast cancer, lung cancer, prostate cancer, kidney cancer, colorectal cancer and skin cancer.

The disclosure will further be illustrated in view of the following figures and examples.

### FIGURE LEGENDS

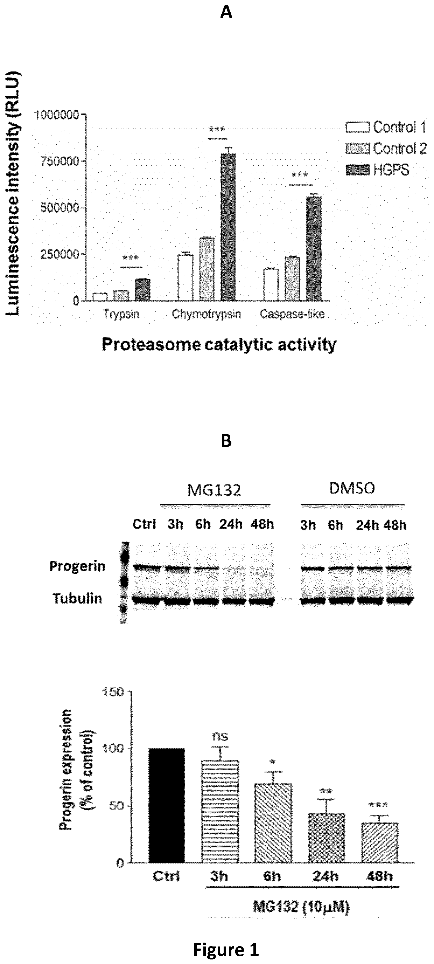

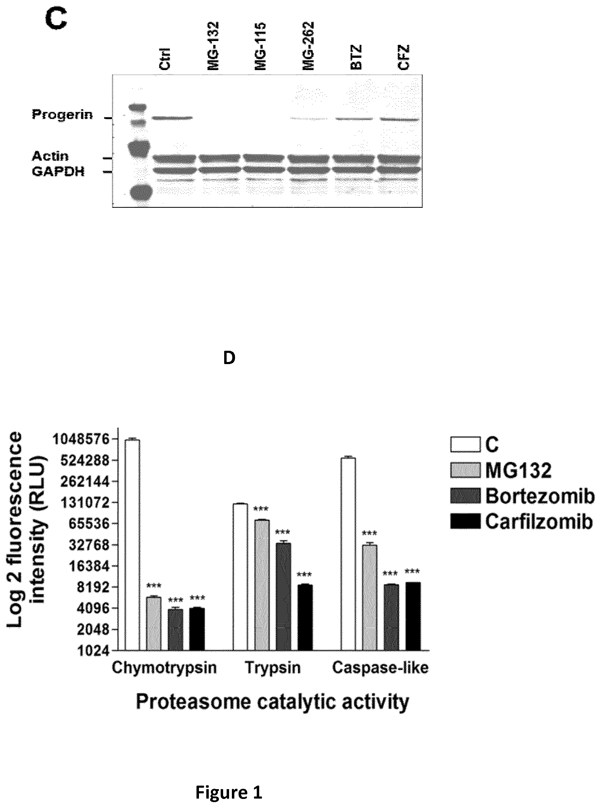

**Figure 1****. MG132, a proteasome inhibitor, reduces progerin levels in HGPS fibroblasts.**
   (A) Significant increase of the proteasome activities (Trypsin: Z-LRR- aminoluciferin, chymotrypsin: Suc-LLVY aminoluciferin and caspase-like: Z-nLPnLD-aminoluciferin) was observed in HGPS fibroblasts (AG01972) relative to control fibroblasts (control 1: 8471 and control 2: 8498) (mean ± SEM, n = 3). Each experiment was performed on cells at the same passage level. Luminescence is determined as relative light units (RLU).
   (B) Upper panels, Western blotting evaluation of progerin and tubulin in whole lysates from HGPS fibroblasts untreated (Ctrl) or treated with 10µ M MG132 or DMSO for 3, 6, 24 and 48h), Lower panels, progerin bands, described in the upper panel, were quantified by ImageJ software and progerin expression levels were normalized to control cells values. (n = 6 ± SEM). *p < .05, **p < .01, ***p < .001, experimental versus control (DMSO-treated cells).
   (C) Western blotting evaluation of progerin, actin and GAPDH in whole lysates from HGPS fibroblasts treated for 48h with DMSO (Ctrl) or 10µ M MG132, MG115, MG262, bortezomib (BTZ) or carfilzomib (CFZ). Inverted picture.
   (D) Proteasome activities (Trypsin: Z-LRR- aminoluciferin, chymotrypsin: Suc-LLVY aminoluciferin and caspase-like: Z-nLPnLD-aminoluciferin) in HGPS fibroblasts (AG01972, AG11498 and 5968) upon 24h MG132 treatment (mean ± SEM, n = 3). Luminescence is determined as relative light units (RLU). C = Control cells treated with DMSO for 24h.

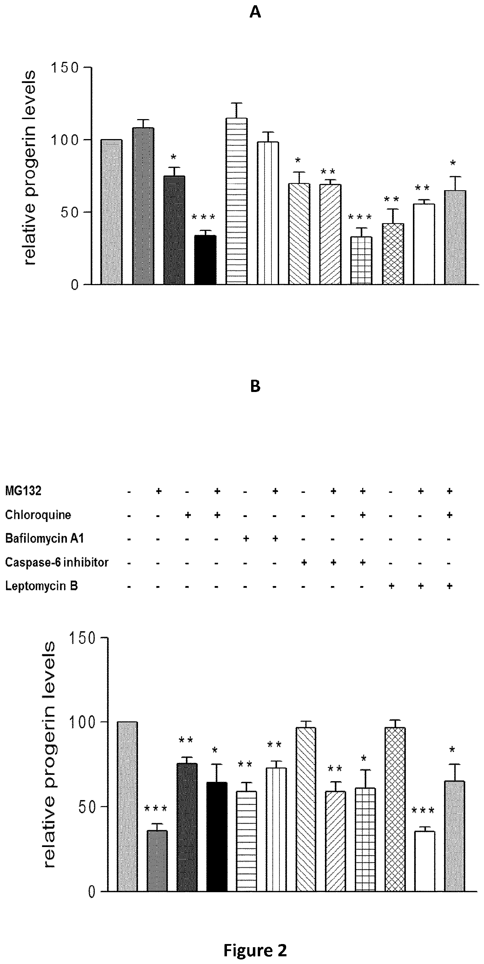

**Figure 2****. Progerin clearance is partially due to autophagy induction.**
   (A) Progerin and GAPDH-specific bands of Western blotting were quantified by ImageJ software and progerin expression levels were normalized to GAPDH values. (n = 6 ± SEM). *P < .05, **P < .01, ***P < .001, experimental versus control (DMSO-treated cells). Progerin levels in whole lysates from, respectively, HGPS fibroblasts untreated (Ctrl) or treated with DMSO (48h), 10µM MG132 (36h and 48h), chloroquine (48h) (1µM, 10µM, 25µ M or 50µ M) or both 10µ M MG132 and chloroquine (1µM, 10µM, 25µM or 50µM) added simultaneously (48h).
   (B) Progerin and GAPDH-specific bands of Western blotting were quantified by ImageJ software and progerin expression levels were normalized to GAPDH values. (n = 4 ± SEM). *P < .05, **P < .01, ***P < .001, experimental versus control (untreated cells). Progerin levels in whole lysates from HGPS fibroblasts treated (+) for 48h with MG132 (10µM), chloroquine (50µM), Bafilomycin A1 (100nM), Caspase-6 inhibitor Z-VEID- FMK (50µM) or Leptomycin B (20ng/ml).
**Figure 3****. MG132 reduces progerin transcripts and splicing factor SRSF-1 protein expression.**
   (A) Downregulation of progerin transcripts in response to MG132. Quantitative real- time PCR analysis of progerin mRNA levels in HGPS fibroblasts treated with 10 µM MG132 relative to DMSO treated cells (Control) (mean ± SEM, n = 3). ***P < .001.
   (B) Upper panels, MG132 reduces splicing factor SRSF-1 expression. Western blotting evaluation of lamin A/C, progerin, Actin, SRSF-1, LC3B-I and LC3B-II in whole lysates from HGPS fibroblasts treated with DMSO (-) (control), MG132 (10 µM), Chloroquine (50 µM), Bafilomycin A1 (100nM) or Pan-caspase inhibitor Z-VAD-FMK. Combined treatments were also performed, as indicated. Lower panels, progerin, SRSF-1 and Actin-specific bands, described in the upper panel, were quantified by ImageJ software. Progerin and SRSF-1 expression levels were normalized to actin values. (mean ± SEM, n = 5).

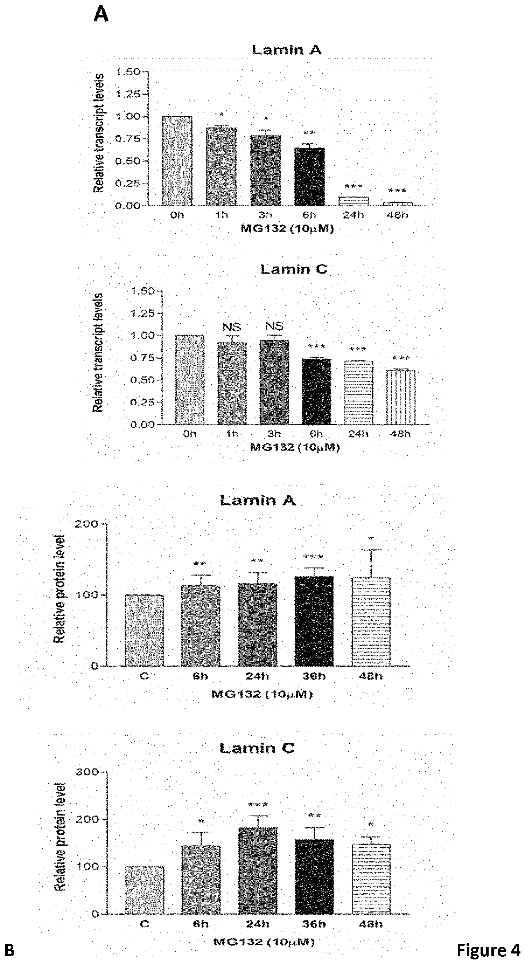

**Figure 4****. MG132 induces a decrease in the mRNA levels but not the protein levels of lamin A/C.**
   (A) MG132 reduces lamin A and lamin C transcript levels. Quantitative real-time PCR analysis of lamin A and lamin C mRNA levels in HGPS fibroblasts treated with 10 µM MG132 (mean ± SEM, n = 3).
   (B) Both lamin A and C protein levels are increased upon HGPS fibroblasts treatment with MG132 (10 µM). A representative Western Blot and the mean values of 3 different experiments are shown. *P < .05, **P < .01, ***P < .001, experimental versus control (DMSO-treated cells).
**Figure 5****. MG132 improves nuclear shape abnormalities in HGPS cell lines.**
   The percentage of normal nuclei (nuclei with a smooth oval shape) and abnormal nuclei (nuclei with blebs, irregular shape, or multiple folds) was calculated by manual blind counting. A representative image and the mean values of 3 different experiments are shown. *P < .05.
**Figure 6****. MG132 reduces progerin levels in patient iPS-Derived MSCs and VSMCs.**
   HGPS fibroblasts, HGPS derived iPS-MSC or iPS-VSMC viability was measured at 24h post treatment with MG132 at the indicated concentrations using CellTiter-Glo Luminescent Cell Viability Assay. Results are reported as viability percentage of MG132-treated cells relative to untreated cells.
**Figure 7****. MG132 reduces progerin levels in** ***Lmna***^{*G609G*/*G609G*} **mice skeletal muscle.**
   (A) Lamin A, progerin, lamin C and SRSF-1 specific bands, corresponding to DMSO-treated left muscle (-) and 1µg/Kg MG132-treated right muscle (+), were quantified by ImageJ software and their expression levels were normalized to GAPDH values.
   (B) The same quantification as in (A) upon 10µg/Kg treatment. *p < .05, **p < .01, MG132-treated versus control untreated cells.

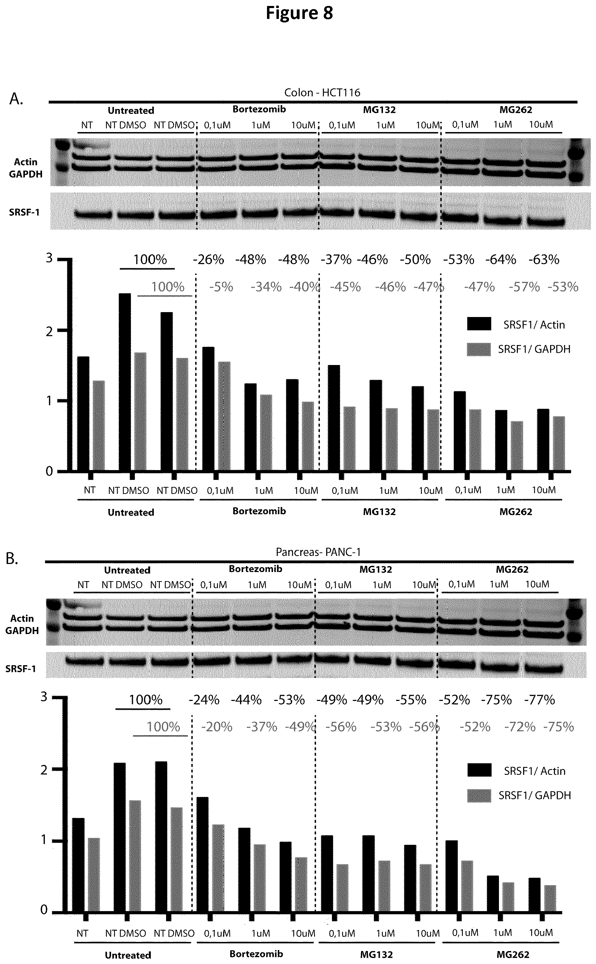

**Figure 8****. Proteasome inhibitors reduce SRSF1 levels in two cultured human adenocarcinoma cell lines.**
   The two cell lines were incubated for 24h with 3 concentrations of proteasome inhibitors diluted in DMSO.
   20 µg of total protein extracts were deposited in each lane.
   The amount of SRSF1 after 24h incubation with the proteasome inhibitors was expressed in percentage of the amount of SRSF1 in control cells incubated with DMSO vehicle only and related to actin (black bins) or GAPDH (grey bins).
   (A) HTC116, colon adenocarcinoma
   (B) PANC-1, pancreas adenocarcinoma.

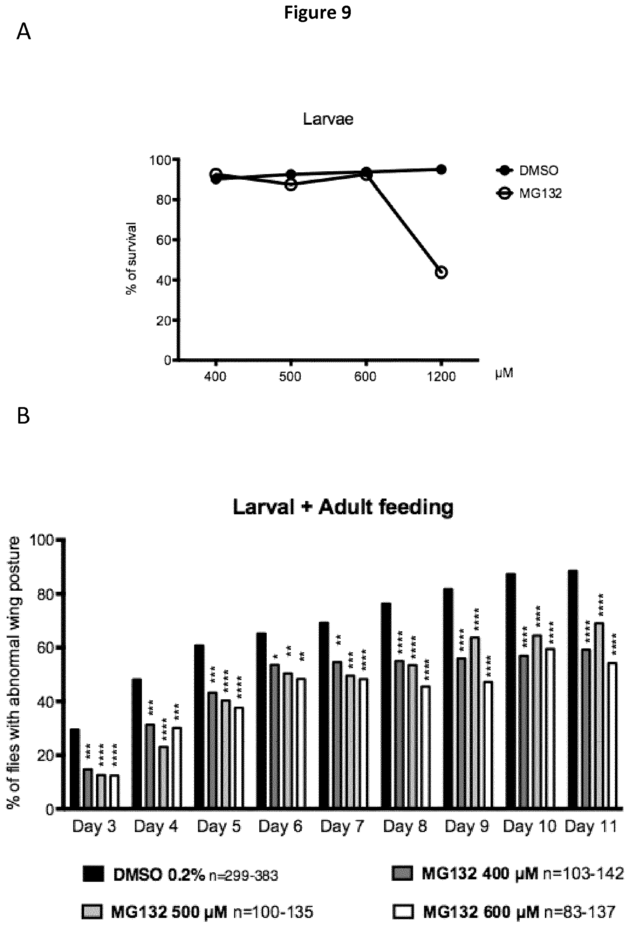

**Figure 9****. Beneficial effect of MG132 in the *Drosophila* OPMD model.**
   (A) The toxicity of MG132 fed to larvae was determined by transferring 80 wild-type first instar larvae into vials containing 5 ml of instant *Drosophila* medium reconstituted either with increasing concentrations of drug in DMSO, or DMSO alone. The graph shows the percentage of flies reaching adulthood at 25°C.
   (B) Quantification PABPN1-17ala-expressing flies (*Act88F-PABPN1-17ala*/+*)* showing wing position defects, either fed with DMSO alone or with three different concentrations of MG132, at 25°C. All concentrations resulted in a decreased number of flies with abnormal wing posture. The numbers of scored flies are indicated. *** *p*-value < 0.001 and ** *p*-value < 0.01 using the chi-square test.

### EXAMPLE 1: Efficient clearance of progerin through autophagy induction and SRSF-1 downregulation under MG132 treatment in Hutchinson-Gilford progeria syndrome

### MATERIAL AND METHODS

### Cell cultures

Human dermal fibroblasts (fibroblasts established from a skin biopsy) from control subjects (8498: 1-year-old male, 8471: 60-year-old male, 731C: 96-year-old male) and patient who carried the HGPS p.Gly608Gly mutation (5968: 5-year-old female) have been prepared and stored according to the regulation by the Biological Resource Center, (CRB TAC), Department of Medical Genetics, Timone Hospital of Marseille (A. Robaglia-Schlupp and K. Bertaux). HGPS fibroblasts (AG01972: 14-year-old female, AG11498: 14-year-old male) were obtained from the Coriell Cell Repository. Cells were cultured in Dulbecco's modified Eagle's medium (Life Technologies) supplemented with 15% fetal bovine serum (Life Technologies), 2 mM L-glutamine (Life Technologies) and IX penicillin-streptomycin (Life Technologies) at 37°C in a humidified atmosphere containing 5% CO2. Fibroblasts were cultured in the presence or absence of MG132 (474790. Merck Chemical LTD), MG115 (SCP0005. Sigma), MG262 (1-120-200. R&D Systems), Bortezomib (S1013. Euromedex), Carfilzomib (S2853. Euromedex), Chloroquine diphosphate crystalline (C6628. Sigma), Bafilomycin A1 (B1793. Sigma), Caspase-6 inhibitor Z-VEID-FMK (FMK006. R&D Systems), Pan-caspase inhibitor Z-VAD-FMK (FMK001.R&D Systems) and Leptomycin B (L2913. Sigma).

### siRNA transfection

For knockdown of SRSF-1, HGPS cells were transfected with 10 nM siRNA using INTERFERin (Polyplus Transfection). HGPS cells were seeded the day before transfection at 100000-200000 cells in 6-well culture vessel. 22 pmoles siRNA were diluted in 200 µl of serum-free DMEM medium, Mixed with 8 µl of INTERFERin reagent. Tubes were vortexed 10 s and incubated 10 minutes at room temperature. The transfection mix was added to the cells in serum containing DMED medium. Medium was changed after 4 hours and cells incubated at 37 °C, 5% CO2 for 48 hours. SRSF-1 siRNA (AM16708) were purchased from Thermo Fisher Scientific.

### Antibodies

Antibodies used in the study included: a Rabbit anti-lamin A/C polyclonal antibody (SC-20681, used at 1:1000 dilution for the Western blot analyzes and at 1:200 for immunofluorescence labeling. Santa Cruz Biotechnology, Inc.); a mouse anti-progerin monoclonal antibody (sc-81611 used at 1:1000 dilution for the Western-blot analyzes and at 1:200 for immunofluorescence labeling. Santa Cruz Biotechnology, Inc.); a rabbit anti-PML polyclonal antibody (sc-9863-R used at 1:200 for immunofluorescence labeling. Santa Cruz Biotechnology, Inc.); a mouse anti-PML monoclonal antibody (sc-966 used at 1:100 for immunofluorescence labeling. Santa Cruz Biotechnology, Inc.); a goat anti-SP100 polyclonal antibody (sc-16328 used at 1:50 for immunofluorescence labeling. Santa Cruz Biotechnology, Inc.); a mouse anti-HP1 monoclonal antibody (2HP-1H5-AS used at 1:100 for immunofluorescence labeling. Euromedex); a rabbit anti-ATRX polyclonal antibody (sc-15408 used at 1:50 for immunofluorescence labeling. Santa Cruz Biotechnology, Inc.); anti-DAXX (ab32140 used at 1:50 for immunofluorescence labeling. Abcam); a rabbit polyclonal anti-CBP antibody (06-297 used at 1:200 for immunofluorescence labeling. Upstate); a mouse anti-ubiquitin antibody (PW8805 used at 1:10 for immunofluorescence labeling. Enzo); a mouse anti-Mono- and polyubiquitinylated conjugates monoclonal antibody (FK2, used at 1:1000 for the Western-blot analyzes. Enzo); a rabbit anti-Fibrillarin polyclonal antibody (ab5821 used at at 1:100 for immunofluorescence labeling. Abcam); a goat polyclonal anti-lamin B1/B2 antibody (sc6217 used at 1:100 for immunofluorescence labeling. Santa Cruz Biotechnology, Inc.); a goat anti-Emerin polyclonal antibody (sc-8086 used at 1:100 for immunofluorescence labeling. Santa Cruz Biotechnology, Inc.); a mouse anti-26S monoclonal antibody (65144 used at 1:1 for immunofluorescence labeling. Progen Biotechnic.); a mouse anti-Nup153 monoclonal antibody (ab93310, used at 1:10 for immunofluorescence labeling. Abcam); a mouse anti-p53 monoclonal antibody (ab26, used at 1:200 for immunofluorescence labeling. Abcam); a rabbit anti-p62 polyclonal antibody (ab37024, used at 1:1000 for the Western-blot analyzes and at 1:100 for immunofluorescence labeling. Abcam); a rabbit anti-LC3B polyclonal antibody (#2775, used at 1:1000 for the Western-blot analyzes and at 1:400 for immunofluorescence labeling. Cell Signaling); a rabbit anti-SQSTM1/p62 polyclonal antibody (#5114, used at 1:1000 for the Western-blot analyzes and at 1:100 for immunofluorescence labeling. Cell Signaling); a rabbit anti-lamin C polyclonal antibody (BP4505, used at 1:200 for immunofluorescence labeling. Acris Antibodies. GmbH); a rabbit anti SRSF1 monoclonal antibody (ab129108, used at 1:1000 for the Western-blot analyzes. Abcam); a mouse anti-Glyceraldehyde-3-Phosphate Dehydrogenase monoclonal antibody (MAB374, used at 1:10000 for the Western-blot analyzes. Merck Millipore); a mouse anti-α-tubulin monoclonal antibody (T6074, used at 1:10,000 for the Western-blot analyzes. Sigma) or a mouse anti-β-actin monoclonal antibody (AC-40, used at 1:10,000 for the Western-blot analyzes. Sigma); a rabbit anti-Sumo2/3 polyclonal antibody (ab-3742, used at 1:1000 for the Western-blot analyzes. Abcam).

### Fluorescence microscopy

Cells collected by centrifugation were spread onto polylysine-coated slides by centrifugation (300 rpm for 5 minutes) with a cytospin (Shandon). Cells were then fixed at room temperature for 10 minutes in 2% (w/v) paraformaldehyde in PBS pH 7.2. Slides were stored at -80°C prior to use. Cells were permeabilized using 100 µL permeabilization buffer (0.5% Triton X-100, 50 mM NaCl, 300 mM sucrose, 20 mM HEPES pH 7.5, 3 mM MgC12) for 3 minutes at RT. Non-specific antibody binding was blocked with 3% Bovine Serum Albumin in PBS (w/v) (PBS-BSA) for 30 minutes. The permeabilized cells were incubated with the primary antibodies for 3h at 37°C. After washing, the cells were then incubated with secondary antibodies (A11001, A11058, Life Technologies; 1/400) for 20 minutes at 37°C. Nuclei were stained with DAPI (50 ng/ml) diluted in Vectashield (Abcys) for 10 minutes at RT. Samples were fixed in 4% paraformaldehyde for 5min. The stained cells were observed either on an Axioplan 2 imaging microscope (Carl Zeiss) or examined through a Nikon inverted microscope attached to a laser confocal scanning system (Leica). All antibodies were tested in individual staining reactions for their specificity and performance. Controls without primary antibody were all negative.

### Quantitation of abnormal nuclear morphology

Fibroblasts from three HGPS patients (AG01972, AG11498, 5968) and two normal individuals (8471, 8498) were cultured with DMEM medium containing 10µM MG132, or the same volume of vehicle (DMSO, 0.025% v/v) for 48h. Cells stained for lamin A/C or DAPI were examined by fluorescence microscopy with an Axioplan 2 imaging microscope (Carl Zeiss). For quantitation of abnormal nuclear morphology, the percentage of normal nuclei (nuclei with a smooth oval shape) and abnormal nuclei (nuclei with blebs, irregular shape, or multiple folds) was calculated by manual blind counting. At least 200 fibroblast nuclei were randomly selected for each cell line and examined in two independent experiments. Results are expressed graphically as the average percentage of the total nuclei counted.

### RNA isolation, reverse transcription, and real-time PCR

Total RNA was isolated using the RNeasy plus extraction kit (Qiagen, Valencia, CA). 1µg of RNA was reverse transcribed using the High Capacity cDNA Reverse Transcription Kit (Applied Biosystems). Real time PCR amplification was carried out with the TaqMan® Gene Expression Master Mix (Applied Biosystems) on a LightCycler 480 (Roche, Germany) using pre-designed primers for GAPDH (Hs00266705_g1), Progerin (F: ACTGCAGCAGCTCGGGG (SEQ ID NO:3) R: TCTGGGGGCTCTGGGC (SEQ ID NO:4) and probe: CGCTGAGTACAACCT (SEQ ID NO:5)), Lamin A (F: TCTTCTGCCTCCAGTGTCACG (SEQ ID NO:6) R : AGTTCTGGGGGCTCTGGGT (SEQ ID NO:7) and probe: ACTCGCAGCTACCG (SEQ ID NO:8)) and lamin C (F: CAACTCCACTGGGGAAGAAGTG (SEQ ID NO:9) R: CGGCGGCTACCACTCAC (SEQ ID NO:10) and probe: ATGCGCAAGCTGGTG (SEQ ID NO:11)) (Applied Biosystems) using the program: UNG incubation at 50°C for 2 minutes, initial denaturation at 95° C for 10 minutes, 40 cycles of amplification: denaturation at 95°C for 15 seconds and annealing at 60°C for 1 minute. All PCR reactions were performed in triplicate. Threshold cycle (Ct) values were used to calculate relative mRNA expression by the 2-ΔΔ^{CT} relative quantification method with normalization to GAPDH expression.

### Western-Blot

Total fibroblast proteins were extracted in 200 µL of NP40 Cell Lysis buffer (Invitrogen, Carlsbad, CA) containing Protease and Phosphatase Inhibitor Cocktail (Thermo Scientific). Alternatively, cells were lysed with urea (8 M urea, 5 mM dithiothreitol, 150 mM NaCl, 50 mM Tris-Cl pH 7.5, protease and phosphatase Inhibitor Cocktail (Thermo Scientific)). Cells were sonicated twice (30 sec each), incubated at 4°C for 30 minutes and then centrifuged at 10,000 g for 10 minutes. Protein concentration was evaluated with the bicinchoninic acid technique (Pierce BCA Protein Assay Kit), absorbance at 562 nm is measured using nanodrop 1000 (Thermo Fisher Scientific Inc.) Equal amounts of proteins (40 µg) were loaded onto 10% Tis-Glycine gel (Criterion™ XT precast gel) using XT Tricine running Buffer (Biorad, USA). After electrophoresis, gels were electro transferred onto nitrocellulose membranes or Immobilon-FL polyvinylidene fluoride membranes (Millipore), blocked in Odyssey Blocking Buffer diluted 1:1 in PBS for 1 hour at room temperature, and incubated overnight at 4°C or 2 hours at room temperature with various primary antibodies. Blots were washed with TBS-T buffer [20 mM tris (pH 7.4), 150 mM NaCl, and 0.05% Tween 20] and incubated with 1:10,000 IR-Dye 800-conjugated secondary donkey anti-goat or IR-Dye 700-conjugated secondary anti-mouse antibodies (LI-COR Biosciences) in Odyssey blocking buffer (LI-COR Biosciences). For IR-Dye 800 and IR-Dye 700 detection, an Odyssey Infrared Imaging System (LI-COR Biosciences) was used. GAPDH, α-tubulin or β-actin were used as a total cellular protein loading control.

### Immunoprecipitation

Immunoprecipitation was carried out using the Dynabeads Protein G Immunoprecipitation kit (Invitrogen). 5 µg of mouse monoclonal anti-Progerin antibody (Abcam) was added to 50 µl (1.5 mg) Dynabeads and rotated at room temperature for 10 minutes. The supernatant was removed and beads-antibodies complex washed in 200 µL PBS with Tween-20 containing 5 mM N-Ethylmaleimide (NEM, Thermoscientific). The supernatant was removed and Dynabeads were incubated with 200 µl cell lysate (200 µg protein) extracted using NP40 Cell Lysis buffer (Invitrogen) containing protease, phosphatase Inhibitor Cocktail (Thermo Scientific) and 5 mM NEM, the tubes were rotated at room temperature for 30 minutes. The beads were washed 3 times according to the manufacturer's protocol. Proteins were eluted with 20 µl elution buffer containing 5 mM NEM. SDS/PAGE and immunoblotting was performed as described above.

### Proteasome activities assays

The proteasome activities were determined in HGPS and control cells by using the Proteasome-Glo™ 3-Substrate Cell-Based Assay System (Promega) that allows to measure the chymotrypsin-like, trypsin-like or caspase-like protease activity associated with the proteasome complex in cultured cells. The Proteasome-Glo™ Cell-Based Reagents each contain a specific luminogenic proteasome substrate. These peptide substrates are Suc-LLVY aminoluciferin (Succinyl-leucine-leucine-valine-tyrosine-aminoluciferin), Z-LRR-aminoluciferin (Z-leucine-arginine-arginine-aminoluciferin) and Z-nLPnLD-aminoluciferin (Z-norleucine-proline-norleucine-aspartate-aminoluciferin) for the chymotrypsin-like, trypsin-like and caspase-like activities, respectively. Cells (10,000 cells/well) were cultured in 100µl/well in a 96-well plate at 37°C, 5% CO2. The plate was allowed to equilibrate to 22°C before 100 µl/well of substrate and luciferin detection reagent was added. The contents of the wells were mixed at 700 rpm using a plate shaker for 2 minutes and incubated at room temperature for 10 minutes. Following cleavage by the proteasome, the substrate for luciferase (aminoluciferin) is released, allowing the luciferase reaction to proceed and produce light. Luminescence is determined as relative light units (RLUs) using a GloMax-Multi Detection System: Luminometer (Promega, USA).

### Measurement of senescence

Senescence was measured with a Beta-Glo Assay kit (Promega), according to the manufacturer's instructions. Luminescence intensity was determined as relative light units (RLUs) using a GloMax-Multi Detection System: Luminometer (Promega, USA).

### Viability and cytotoxicity assays

Viability and cytotoxicity were measured with a MultiTox-Fluor Multiplex Cytotoxicity Assay (Promega), according to the manufacturer's instructions. The MultiTox-Fluor Assay simultaneously measures two protease activities: one is a marker of cell viability using a fluorogenic, cell-permeant peptide substrate (glycyl-phenylalanylamino fluorocoumarin; GF-AFC), and the other is a marker of cytotoxicity (bisalanyl-alanyl-phenylalanyl-rhodamine 110; bis-AAF-R110). The live- and dead-cell proteases produce different products, AFC and R110, which have different excitation and emission spectra, allowing them to be detected simultaneously. Results are provided in relative fluorescence units (RFU) measured using a GloMax-Multi Detection System: Luminometer (Promega, USA). Data are representative of at least three independent experiments done in triplicate.

### Proliferation assay

Cells proliferation rate was measured with a CellTiter 96 Aqueous One Solution Cell Proliferation Assay (Promega), according to the manufacturer's instructions. Absorbance was monitored with a GloMax-Multi Detection System: Luminometer (Promega, USA).

### Fibroblasts reprogramming

Generation of induced pluripotent stem cells (iPS) by reprogramming was carried out using fibroblasts isolated from HGPS patient biopsies performed in the Assistance Publique Hôpitaux de Marseille (13-8243) or provided by Coriell Institute (Camden, USA) (AG01972). Cell lines were reprogrammed to iPSC using Yamanaka's original method based on retrovirus-mediated transfection of four transcription factors, namely Oct3/4, Sox2, c-Myc, and Klf4 (*41*). The iPSC lines were amplified up to the 15th passage before differentiation.

### Pluripotent stem cells culture and differentiation

HGPS iPSC were grown on STO mouse fibroblasts, inactivated with 10 mg/ml mitomycin C, seeded at 30,000/cm2 and grown as previously described (*42*). For differentiation, iPSC were differentiated into mesenchymal stem cells (MSC-iPSC) using directed protocols for differentiation previously published (*43-45*). VSMC were obtained from iPSC by fluorescent-activated cell sorting (FACS) separation of CD31+ cells (*46*).

### Mice

Knock-in mouse model (*Lmna*^{*G609G*/*G609G*}) carrying the c.1827C>T (p.Gly609Gly) mutation in the endogenous *Lmna* gene, corresponding to the human HGPS mutation c.1824C>T (p.Gly609Gly) have been described previously (*31*). MG132 (1 or 10 µg/Kg body weight) was injected under isoflurane anesthesia into the right gastrocnemius muscle of 8 month-old homozygous knock-in mice (*Lmna*^{*G609G*/*G609G*}) 3 times per week for 2 weeks. Contralateral muscle served as control (DMSO). MG132 was dissolved in dimethyl sulfoxide (DMSO) and then diluted in 100 µl sterile phosphate-buffered saline (PBS) for intramuscular injection. Neither vehicle alone nor MG132 treatment produced any apparent damage or stress responses in mice. Animal experiments have been carried out in compliance with the European guidelines for the care and use of laboratory animals (EU directive 2010/63/EU) and in accordance with the recommendations provided by the guide for the care and use of the laboratory animals of the French national Institute for Health and Medical Research (INSERM) and the ethical committee of Aix-Marseille University.

### Statistical analyzes

Statistical analyzes were performed with the GraphPad Prism Software. Differences between groups were assayed using a two-tailed Student's t-test using GraphPad Prism. In all cases the experimental data were assumed to fulfil t-test requirements (normal distribution and similar variance); in those cases where the assumption of the t-test was not valid, a non-parametric statistical method was used (Mann-Whitney test). A P value less than .05 was considered as significant. Error bars indicate the standard error of the mean.

### RESULTS

### Identification of thread-like PML-NBs as novel biomarkers in HGPS cell lines

The classical hallmark of HGPS cells is the accumulation of progerin, the inventors therefore investigated the subcellular localization of progerin in fibroblasts of three patients affected with HGPS (AG11498, AG01972, 5968). Immunostaining revealed an extensive accumulation of intranucleoplasmic progerin punctuate dots and fibrous aggregates at later passages in culture (data not shown).

Since PML-NBs have been proposed to function as sites of sequestration of misfolded proteins targeted for proteasomal degradation (*47*) and have been implicated in the control of cellular senescence (*48*), the inventors hypothesized that these organelles might be involved in progerin accumulation or degradation. The inventors explored the localization of PML protein in fibroblasts of three patients affected with HGPS (AG11498, AG01972, 5968) and fibroblasts of two healthy subjects (8471, 8498). At early passages (passages 9-15), the PML protein distribution was similar in patients and controls, with PML-NBs appearing as classical punctuate structures (10-30 bodies per cell of 0.5 µm diameter). At late passages (passages 16-26), the number of unusual spherical and thread-like PML-NBs increased in HGPS nuclei, relatively to the cell doubling in culture. In contrast, abnormal PML-NBs were very rarely detected in normal control fibroblasts, either in similarly or in more aged cultures (passages 29-33) (data not shown).

The inventors then accessed the composition of PML-NBs. Double immunostaining with anti-PML antibody and antibodies against classical components of PML-NBs (SP100, HP1, DAXX, CBP and ATRX) performed on HGPS fibroblasts at late passage, showed that such structures have a composition similar to that of conventional PML- NBs and may have similar functions (data not shown).

Therefore, thread-like PML-NBs may be considered as novel biomarkers in HGPS cell lines.

### PML-NBs are progerin-accumulating organelles in HGPS fibroblasts nuclei

To evaluate a possible correlation between the presence of thread-like PML structures and that of progerin, the inventors performed double immunostaining with anti-PML and anti-progerin antibodies on HGPS fibroblasts. Interestingly, progerin colocalizes with PML. Using confocal microscopy, the inventors showed that PML-NBs contain progerin (data not shown).

To determine whether other nuclear lamina-resident proteins, in addition to progerin, have the capacity to be targeted to the PML-NBs, the inventors tested the subcellular localization of lamins A, C, B1, B2, Nup-153 and emerin. Among the proteins explored, only B-type lamins (B1 and B2) were also included within spherical and thread-like PML-NBs structures. The projections of confocal planes and 3D visualization of PML within the nucleus indicate that thread-like PML-NBs are inside the nucleoplasm (data not shown). In addition, calreticulin, which localizes into the lumen of the endoplasmic reticulum or the nuclear envelope, has never been observed within PML-NBs (data not shown). This suggests that PML-NBs do not correspond to an invagination of the nuclear envelope in which may be anchored progerin, B-type lamins or emerin.

### MG132, a proteasome inhibitor, decreases progerin levels in HGPS cells

Unlike the cytosol, where three main complementary proteolytic systems mediate protein degradation, i.e autophagy, the ubiquitin proteasome system (UPS) and caspases, in eukaryotic cells nuclei only UPS and caspases operate. It is believed that nuclear proteasome-mediated protein degradation occurs within distinct nucleoplasmic foci (*47*). These proteolytic nuclear subdomains represent a subset of the PML-NBs where ubiquitin, proteasomes and SUMO conjugates are concentrated (*49-52*). The presence of ubiquitin and 26S proteasome together with progerin into the thread-like PML structures, strongly supports that they might be involved in the degradation of proteins and in particular progerin via the UPS.

To assess whether proteasome activity levels could influence progerin accumulation in HGPS cells, the inventors measured, using specific fluorogenic substrates, the three distinct proteasome catalytic activities: chymotrypsin, trypsin and caspase-like activity. HGPS cells exhibit increased activities of the three proteasome enzymatic activities as compared to either similarly or more aged healthy subjects (Figure 1A). This indicates that UPS system may reach a saturation point at which the capacity to degrade progerin aggregates is exceeded. Indeed, given that HGPS patients fibroblasts display elevated ROS (*53*), oxidative stress might lead to saturation of the proteasome resulting from an excessive amount of misfolded, oxidatively damaged and ubiquitinated proteins. It has been previously described a significant decrease in the protein-degrading capability of proteasomes in HGPS fibroblasts; an impairment that occurs, in part, as a consequence of a decreased number of subunits required to assemble the proteolytic complex as well as high levels of ROS and oxidized proteins (*54*). Altogether, these results indicate that progerin is trapped within PML-NBs and not naturally degraded through the ubiquitin proteasome system (UPS).

To determine whether proteasome inhibitors influence the level of progerin, the inventors performed a time course treatment with 10 µM MG132 on HGPS cell lines and the levels of progerin were investigated by Western-Blot. Unexpectedly, MG132 induced a decrease of progerin levels from 6h until 48h of MG132 treatment. When normalized with a tubulin control, quantification of protein by Western blot revealed a ∼31%, 58% and 65% reduction in progerin amounts at 6h, 24h and 48h respectively, in MG132-treated HGPS cells as compared to DMSO-treated HGPS cells (Figure 1B).

### Peptide aldehyde (MG132), and peptide boronate (MG262) proteasome inhibitors but not other boronate analogs (Bortezomib and Carfilzomib) elicit progerin clearance while they exhibit different inhibition in proteasome enzymatic activity

To confirm or rule out the involvement of the proteasome inhibition in progerin clearance, the inventors tested several proteasome inhibitors in the same conditions. MG115 (Z-Leu-Leu-nVal-al), another aldehyde proteasome inhibitor, initially thought as less potent than MG132, was found as having the same efficacy in suppressing progerin accumulation (Figure 1C). The boronate analogue MG262 (Z-Leu-Leu-Leu- boronate) was less effective despite its greater affinity for the active sites of the proteasome. MG262 is more potent than the aldehyde MG132 (*55*), and the inventors thus expected that MG262 would be efficient at lower concentrations than MG132 to achieve the same effect. However, the inventors found that MG262 induced progerin clearance in HGPS cells only at equivalent doses to that of MG132 (data not shown). These findings are consistent with the known differential action of MG132 and MG262 on caspase and proteasome enzymatic activities (*56*).

The inventors next tested the effect of bortezomib and carfilzomib, two FDA approved (treatment of multiple myeloma) proteasome inhibitors, on progerin clearance. Unlike aldehydes MG132 and MG115, these two molecules do not induce progerin clearance as progerin expression is maintained as in HGPS controls (Figure 1C and further data not shown). This inefficiency is probably related to their distinct effect on proteasome enzymatic activities in HGPS cell lines (Figure 1D). However, these findings suggest that the MG132 effect on progerin clearance is not only mediated by its known action on the proteasome.

### Progerin clearance is partially due to autophagy induction

Besides the ubiquitin-proteasome system, another major route for intracellular protein degradation is the autophagy-lysosome pathway. The observed progerin decrease upon proteasome inhibition raised the question of how this clearance can occur and further suggests that progerin could be a substrate for autophagic degradation, especially since it has been shown that proteasome inhibition resulted in a compensatory enhancement of autophagy (*57-59*). In order to assess whether the macroautophagy process was involved in MG132-enhanced progerin clearance, the inventors inhibited autophagy by using either chloroquine a lysosomotropic agent that directly alter the acidic pH of lysosomes, or bafilomycin A1, an inhibitor of lysosomal proton pump. Treatment of MG132 exposed HGPS fibroblasts with high-dose chloroquine or bafilomycin A1 partially inhibited the effect of MG132 and induced partial progerin re-expression (Figure 2), suggesting that the increased clearance of progerin observed with MG132 treatment is mediated in part by the autophagic-lysosomal pathway. Strikingly, treatment with chloroquine or bafilomycin A1 alone at high doses also caused reduced expression levels of progerin (Figure 2). Indeed, it has been reported that inhibition of autophagy leads to increased proteasome activities and upregulation of proteasomal subunits in cultured human colon cancer (*60*). However the three proteasome activities are not modified upon treatment of HGPS cells with these two inhibitors. These findings thus suggest that proteasome activation is not involved in the reduction of progerin levels induced by chloroquine or bafilomycine A1. LC3B was used to evaluate the autophagy level as the conversion of LC3-I to LC3-II by lipidation provides an indicator of autophagic activity. As expected, LC3B-II levels are increased following MG132 treatment, concomitantly with the decrease of progerin levels (Figure 2A). The incomplete progerin re-expression after UPS and autophagy simultaneous blockade suggests that MG132 efficiency on the progerin clearance is mediated, in addition to autophagy, by another proteolytic concomitant activity.

Caspase-6 is widely regarded to be responsible for lamins A and C cleavage during apoptosis. Lamin A/C but also progerin exhibit a conserved VEID caspase 6 clivage site (*61-64*) located in the non-helical linker region L12 at position 227-230. To investigate the involvement of caspase-6 in MG132-induced progerin clearance, the inventors treated HGPS cells for 48h with a combination of MG132 and caspase-6 inhibitor. Again, progerin level remained below the HGPS control either in the presence or absence of chloroquine (Figure 2B). Otherwise, since progerin has a leucine-rich nuclear export signal (NES), a possible CRM1-mediated progerin export to the cytoplasm was tested using leptomycin B, inhibiting the CRM1-dependant nuclear export of proteins containing a leucine-rich NES (*65*). It turned out that leptomycin B does not inhibit MG132 induced progerin clearance. Since progerin cannot be degraded in the nucleoplasm by proteasome when inhibited by MG132, its clearance could thus be explained either by its degradation through an alternative route, or by downregulation of neosynthesized protein or by a leptomycin B-insensitive efflux of nucleoplasmic progerin. Indeed, the exit to the cytoplasm through nuclear envelope disruptions of nuclear macromolecular or PML-containing complexes has been already shown in laminopathies (*66*).

### Progerin accumulates in autophagic vacuoles upon proteasome inhibition using MG132

It has been reported that the MG132 can lead to PML-NBs component proteins being translocated into the nucleolus [35,36]. Other recent findings have illustrated a dynamic relationship between the nucleolus and PML-NBs [37-39]. To investigate subcellular progerin localization upon MG132 treatment, the inventors carried out a time course indirect immunofluorescence using progerin and PML antibodies on HGPS cell lines treated with MG132 (10 µM) for 6h and 24h. 24h treatment with MG132 induces the formation of large progerin and PML intranuclear *foci* (data not shown). Whether in fibroblasts from healthy subjects or those of HGPS patients, analyzes using antibodies against fibrillarin, which localizes to the dense fibrillar component of the nucleolus, showed overlap with PML and Emerin (data not shown). p53 has been reported to localize with inactivated proteasomes to intranucleolar regions distinct from the ribosome production compartments when cells were treated with MG132 (*67*). These observations are consistent with our results as we also observed p53 to be localized to the nucleolus. Under the same conditions, the inventors have shown that lamins B1/B2, 26S proteasome subunit and ubiquitin colocalizes with PML in the nucleolus. Therefore, nucleolus might function as an alternative sequestration site that immobilizes and temporarily stores accumulated progerin when proteasome-dependent degradation is blocked, as the inventors detected PML and LC3B cytosolic colocalization upon 24h MG132 treatment (data not shown). While, during this treatment period, progerin mainly remained localized within the nucleolus (data not shown), after 48h, progerin aggregates were detected into the cytosol where the inventors demonstrated its co- localization with LC3B (data not shown). To further investigate the type of cytosolic progerin-containing aggregates, the inventors explored other markers of autophagic vacuoles such as p62, a known autophagy substrate and LAMP-2, a lysosomal membrane protein. Double immunofluorescence labeling with progerin and p62 or LAMP-2 antibodies confirmed that cytosolic progerin accumulated in p62 and LAMP-2-positive vesicles after 48h MG132 exposure of HGPS cell lines. Taken together, these results indicate that progerin accumulates into autophagic vacuoles upon MG132 treatment and thus undergoes autophagic degradation in the cytoplasm.

### MG132 downregulates prelamin A aberrant mRNA splicing

Since the blockade of all main degradation pathways was not able to restore the progerin level as compared to HGPS control cells, the inventors further evaluated if MG132 could have an effect at the transcriptional level on lamins and progerin. Thus, the inventors performed quantitative reverse transcription-polymerase chain reaction (qRT-PCR) assays to quantify progerin transcript levels in MG132 treated HGPS and control cells. As shown in Figure 3A, MG132 treatment almost extinguished progerin mRNA expression in a time dependent manner, indicating its dual effect on progerin at both the protein and transcriptional level.

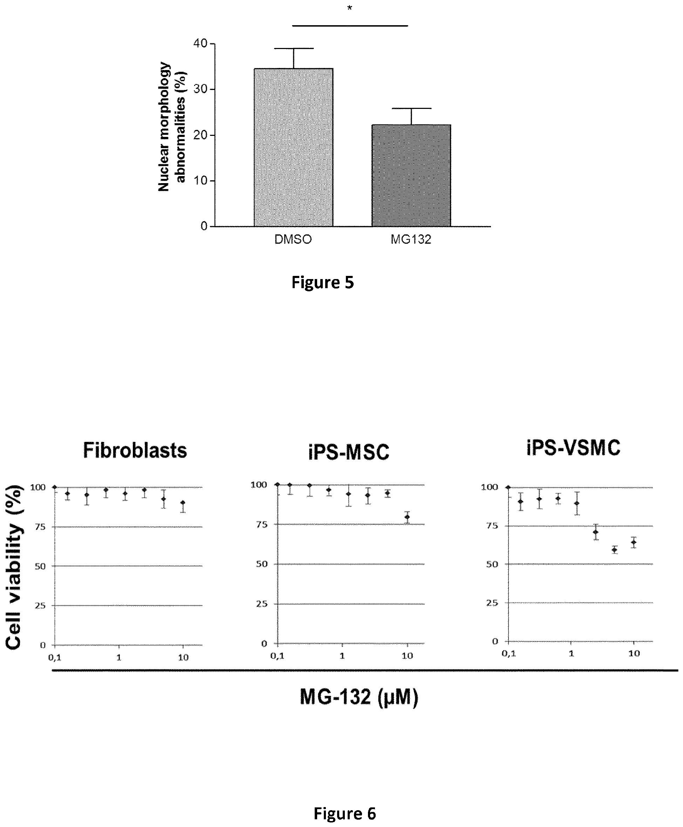

It has been previously shown a regulatory role of a subset of serine-arginine (SR)-rich proteins, including serine-arginine rich splicing factor-1 (SRSF-1) on the recognition of the 5' Splice Site (5'SS) of *LMNA* exon 11 and the production of lamin A and progerin from c.1824C>T mutated *LMNA* alleles (*32*). Furthermore it has been shown that T cell stimulation with MG132 actively induces the degradation of SRSF-1 and that caspase-3 contributes to the regulation of SRSF-1 protein expression during T cell activation (*68*). Given the partial re-expression of progerin in the presence of autophagy inhibitors and the concomitant decreased expression level of progerin mRNA after MG132 treatment, the inventors hypothesized that MG132 effect could also be mediated by a decrease of SRSF-1 expression and therefore, the splicing of progerin pre-mRNA and the production of progerin. Interestingly, the inventors demonstrate that 48h treatment of HGPS fibroblasts with MG132 resulted in a significant decrease of SRSF-1 levels concomitantly with the decrease of progerin levels (Figure 3B). To exclude that upon MG132 addition, progerin may become insoluble and not completely extracted form aggregates, the inventors solubilized the cells with urea. This experiment shows that progerin reappears after 72 h of treatment. Thus a longer term effect on progerin clearance may require retreatment after 48 h. Towards exploring whether mechanisms involving caspases play a role in SRSF-1 expression, the inventors added the pan-caspase inhibitor (VAD) during HGPS cells treatment and observed a rescue of SRSF-1 protein expression, concomitantly with an increase of progerin that slightly reaches the control levels. It is also of great interest to note that higher progerin re-expression was observed by using a combination of MG132, pan-caspase inhibitor and chloroquine. Interestingly, the inventors observed that both autophagy inhibitors (chloroquine and bafilomycin A1) are also able to decrease SRSF-1 levels, which could explain the decrease of progerin levels observed upon their treatment. Bafilomycin A1 effect on reducing SRSF-1 levels has already been described indeed in activated T cells (*68*). To better understand the involvement of SRSF-1 in regulating the expression of progerin, the inventors treated HGPS cells with small-interfering RNAs (siRNAs) to transiently deplete SRSF-1 cell content. The results presented in Figure 5d show that siRNA knockdown of SRSF-1 greatly reduces progerin levels, evidencing the important role of this splicing factor in MG132-induced progerin clearance.

Taken together, the results indicate that autophagy induction, caspases and splicing mechanisms through SRSF1 extinction play converging roles towards progerin clearance under MG132 treatment.

Since SRSF1 is involved in the control of the lamin A/progerin splicing switch, the inventors hypothesized that MG132 modulates lamin A transcript expression. To test this hypothesis, the inventors used qRT-PCR to explore the mRNA levels on HGPS cells following MG132 treatment. Analyzes of MG132 time-course qRT-PCR revealed a significant decrease of lamin A transcripts while a much less pronounced decrease of lamin C transcript levels was observed (Figure 4A). The observed reduction of lamin A/C mRNA prompted the inventors to evaluate their corresponding protein levels upon proteasome inhibition. As shown in Figure 4B, the inventors found that both lamin A and C protein levels are increased during 48 h treatment with MG132.

### MG132 reduces progerin levels in patient iPS-derived MSCs and VSMCs

To determine whether MG132 also influences the level of progerin in other cell lineages, the inventors generated iPSC from HGPS patients' fibroblasts. Mesenchymal Stem Cells (MSC) and Vascular Smooth Muscle Cells (VSMC) derived from HGPS iPSC presented the commonly described abnormalities of HGPS fibroblasts, namely, loss of proliferation capacities, premature senescence and nuclear blebbing (*69, 70*). The inventors performed immunostaining of MG132-treated VSMC with progerin antibody.

MG132 treated VSMC led to a reduction of progerin significantly starting at 1.25µM (data not shown). In parallel, the inventors investigated progerin and SRSF-1 levels upon MG132 treatment and compared HGPS fibroblasts, iPS-MSC and iPS-VSMC. This revealed that, in all the tested cell lines, progerin and SRSF-1 levels were decreased by MG132 treatment (data not shown). Observed progerin clearance is not linked to increased apoptosis or cell death, as this is the case for the other FDA approved proteasome inhibitors, since viability test showed that the doses used in this study do not induce significant mortality in HGPS fibroblasts, iPS-MSC and iPS-VSMC (Figure 6), indicating a very low toxicity and a strong potential of MG132 as a therapeutic agent for progeria.

### MG132 reduces progerin levels in Lmna^{G609G/G609G} mice muscle.

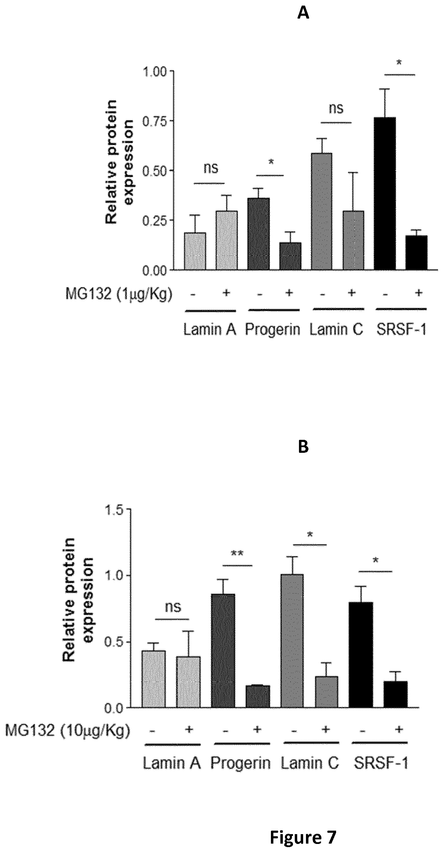

Because MG132 induces an almost complete clearance of progerin in various cell lines, it was of interest to investigate its effect *in vivo.* The inventors therefore tested whether intramuscular injection of MG132 might also enhance progerin clearance in our Knock-in mouse model *(Lmna*^{*G609G*/}*^{G609G})* carrying the c.1827C>T (p.Gly609Gly) mutation in the endogenous *Lmna* gene (*31*), corresponding to the human HGPS mutation c.1824C>T (p.Gly608Gly). As shown in Figure 7, 1µg/Kg treatment with MG132 induces a significant decrease of progerin level in the treated muscle compared to the untreated collateral muscle. Under these conditions a small increase in lamin A and a decrease of lamin C were observed while these changes are not significant. At 10µg/kg body weight, MG132 injection also results in a significant decrease of progerin levels but is accompanied by a decrease in lamin C. However, the levels of lamin A remain constant or increase slightly which should compensate the Lamin C decrease as previously shown in *Lmna*^{LAO/LAO} mice which were healthy despite a complete absence of lamin C (*71*).

These data provide evidence that in fibroblasts from HGPS patients, progerin is sequestered in PML-NBs where the proteasome and ubiquitinated proteins are located. Proteasome inhibition using MG132 induces both caspase-mediated reduction of SRSF-1 expression levels and autophagy-induced progerin degradation that occurs after nucleolar translocation of progerin and its subsequent export into the cytosol.

The results reported herein provide an encouraging strategy for treating children affected with Progeria, based on the progerin clearance upon MG132 treatment. The observed specificity on progerin, sparing A-type Lamins, further supports the idea that beside typical Progeria, prelamin A deficient associated diseases might benefit the same treatment. Finally, considering the activity of proteasome inhibitors of MG aldehyde family (MG132 and MG115 in particular) on SRSF-1 downregulation, this family of molecules could have beneficial impacts on physiological ageing during which progerin accumulates, as well as on diseases involving the activation of cryptic splice sites used by SRSF-1 or diseases exhibiting SRSF1 overexpression such as some cancers.

### EXAMPLE 2 : Proteasome inhibitors downregulate SRSF-1 in human adenocarcinoma cell lines

### MATERIAL AND METHODS

### Cell cultures

Six adenocarcinoma human cell lines (lung: A549, HCC827 ; colon: HCT116, HT-29 ; pancreas : PANC-1, Mis PaCa-2) were obtained from ATCC, cultured as required and amplified (passages 50 to 60) in order to get vials containing 5 x 10⁵ to 10⁶ cells/vial that were frozen in DMSO and stored at -80°C before further use. Cell viability was checked as described above.

### Proteasome inhibitor treatment

MG132 (474790. Merck Chemical LTD), MG262 (1-120-200. R&D Systems) and Bortezomib (S1013. Euromedex) were incubated for 24 h at 3 concentrations 0.1, 1 and 10µM in DMSO. Control cells were incubated in DMSO vehicle only.

### Antibodies

Antibodies against SRSF1, lamin A/C, progerin, actin and GAPDH (used as loading controls) have been described in example 1.

### Western-Blot and image analysis protocols have been detailed in example 1 above

### RESULTS

All the studied adenocarcinoma cell lines expressed SRSF-1, with a major band of ∼ 32 kDa corresponding to ASF2 isoform (whose intensity was measured) and two minor bands, ∼ 28 kDa being the ASF1 isoform of SRSF-1 and a band of ∼ 60 kDa being probably a dimer. When 40 µg of proteins were deposited, the SRSF-1 band intensity was twice the intensity measured in lanes containing 20 µg of deposited proteins.

DMSO vehicle increased the amount of SRSF-1 (Figure 8). MG262, MG132 and Bortezomib induced a decrease in the amount of SRSF-1, from -20% to -77%, exhibited a dose-response effect, the inhibitor efficacy increasing from Bortezomib to MG132 then MG262. Moreover, even in the same range, data were different depending upon the loading charge control used, actin or GAPDH.

All the cell lines studies evidenced various amounts of lamin A/C but never expressed progerin.

These results confirmed those obtained in HGPS cells using MG132 and MG262 regarding SRSF1, whereas Bortezomib exhibited a different effect in HGPS cells and in cancer cells.

### EXAMPLE 3 : Proteasome inhibitor MG132 has a positive effect on Oculopharyngeal muscular dystrophy (OPMD) in the Drosophila model

### Introduction

Oculopharyngeal muscular dystrophy (OPMD) is an autosomal dominant, late onset, muscular dystrophy that usually starts in the fifth or sixth decade, and is characterized by progressive eyelid drooping (ptosis), swallowing difficulties (dysphagia) and proximal limb weakness. OPMD is a rare disease (1/100 000 in Europe), with a world-wide distribution; it is the most common muscular dystrophy in Bukhara Jews (1/600) and in Quebec (1/1000) (Reviews: (*72, 73*)). There is no pharmacological treatment for this disease at the moment; surgery can be carried out to improve swallowing and to lift drooping eyelids.

The gene responsible for OPMD has been identified as the gene encoding the poly(A) binding protein nuclear 1 (PABPN1). In OPMD patients, *PABPN1* has an expansion of a GCG repeat encoding a polyalanine tract located in the N-terminus of the protein (*74*). The polyalanine expansion is moderate as 10 alanines are present in the normal protein, and are expanded to 12 to 17 alanines in the mutant forms of the protein.

A pathological hallmark of OPMD is nuclear inclusions in muscle fibers, which are composed of tubular filaments and contain mutant insoluble PABPN1, as well as Hsp70, ubiquitin and subunits of the proteasome (*75-78*). Several RNA binding proteins and the type I arginine methyl transferases, PRMT1 and PRMT3, known to methylate PABPN1, are also recruited in nuclear inclusions (*78-80*)*.* Polyalanine expansions in PABPN1 induce misfolding and aggregation into inclusions resulting from a slower turnover of the mutant protein.

PABPN1 has a function in nuclear polyadenylation, an mRNA processing reaction leading to the formation of the poly(A) tail at the 3' end of mRNAs. PABPN1 binds to nascent poly(A) tails during this reaction and has two roles: stimulation of poly(A) polymerase, the enzyme that synthesizes the poly(A) tail, and control of poly(A) tail length (*81-84*). PABPN1 is also involved in the control of cytoplasmic mRNA poly(A) tails in early embryos (*81*), in poly(A) RNA decay or export from the nucleus (*85*), in the regulation of alternative poly(A) site choice (*86*, *87*), and in the turnover of long non-coding RNAs (lncRNAs) (*88*).

A *Drosophila* model of OPMD has been developed in which alanine expanded mammalian PABPN1 (PABPN1-17ala) is specifically expressed in *Drosophila* muscles (*89*, *90*). These models recapitulate the disease characteristics, namely progressive muscle weakness and degeneration, and formation of PABPN1 nuclear aggregates. The relevance of the *Drosophila* models has been validated by the fact that the molecular mechanisms identified as participating in OPMD in *Drosophila* have been confirmed in OPMD patient biopsies (*91, 91*). *Drosophila* models have been useful in identifying potential active drugs for OPMD, in particular anti-aggregation drugs (*92*).

### Materials and Methods

Drug supplemented food was prepared as follows. Instant *Drosophila* medium (Carolina Biological Supply Company) was reconstituted in each vial with a solution of 1% yeast in water, supplemented with either increasing concentrations of drug solubilized in DMSO (dimethyl sulfoxide), or DMSO alone. MG132 was purchased from Santa Cruz Biotechnology (SC-201270). Each vial contained 5 ml or 2.5 ml of reconstituted medium, to raise larvae or adults, respectively. Individuals were fed with the drug from larval stages. 70 to 80 first instar larvae were transferred per vial and developed in the same vial up to adulthood; adults were transferred in a new vial with the same concentration of drug. To determine drug toxicity, 80 first instar larvae were transferred into vials containing drug- or DMSO alone-supplemented medium. Individuals reaching adulthood were scored. Abnormal wing posture was quantified by collecting adult males at birth, pooling five males per vial and scoring abnormal wing position each day by direct observation of the flies through the vials, without anesthesia.

### Results

Transcriptomic analyses have shown a general deregulation of the Ubiquitin-proteasome system that is conserved in *Drosophila* and mouse models of OPMD and in patient biopsies (*20*). In addition, proteasome activity is upregulated in muscles, in the mouse model of OPMD and this correlates with muscle atrophy (*93*). These results led the inventors to propose that UPS deregulation might participate in OPMD through increased protein degradation that would lead to muscle atrophy. The inventors thus tested if reducing proteasome activity using MG132 might be beneficial for OPMD in the *Drosophila* model. The inventors used the *Drosophila* model in which PABPN1-17ala is expressed constitutively in adult indirect flight muscles from the *Act88F-PABPN1-17ala* transgene (*90*), to assay the effect of MG132 *in vivo.* MG132 was administered orally in the food at three concentrations 400, 500 and 600µM from larval stages. Survival tests showed that 600 µM MG132 (in 0.2% DMSO) in the fly medium is not detrimental for viability (Figure 9A). PABPN1-17ala expression in indirect flight muscles leads to abnormal wing posture resulting from affected muscle function and muscle degeneration (*89*). Wing posture defects were recorded from Day 3 to Day 11 of adulthood. The effect of MG132 was quantified by recording the number of Act88F-PABPN1-17ala-expressing flies with abnormal wing posture. All three MG132 concentrations showed a beneficial effect since they resulted in a significant decrease in the number of flies with abnormal wing posture compared to DMSO alone (Figure 9B). Therefore, a proteasome inhibitor, MG132, improved a genetic myopathy, OPMD, characterized by the nuclear accumulation of toxic protein aggregates.

### REFERENCES

1. R. C. Hennekam, Hutchinson-Gilford progeria syndrome: review of the phenotype. American journal of medical genetics. Part A 140, 2603-2624 (2006).
2. M. A. Merideth et al., Phenotype and course of Hutchinson-Gilford progeria syndrome. The New England journal of medicine 358, 592-604 (2008).
3. A. De Sandre-Giovannoli et al., Lamin a truncation in Hutchinson-Gilford progeria. Science 300, 2055 (2003).
4. M. Eriksson et al., Recurrent de novo point mutations in lamin A cause Hutchinson-Gilford progeria syndrome. Nature 423, 293-298 (2003).
5. P. Cau et al., Nuclear matrix, nuclear envelope and premature aging syndromes in a translational research perspective. Seminars in cell & developmental biology, (2014).
6. P. Scaffidi, T. Misteli, Lamin A-dependent nuclear defects in human aging. Science 312, 1059-1063 (2006).
7. D. McClintock et al., The mutant form of lamin A that causes Hutchinson-Gilford progeria is a biomarker of cellular aging in human skin. PloS one 2, e1269 (2007).
8. H. Takeuchi, T. M. Runger, Longwave UV Light Induces the Aging-Associated Progerin. J Invest Dermatol 7, 71 (2013).
9. M. Hosseini et al., Premature aging features rescue by inhibition of NADPH oxidase activity in XPC deficient mice. Journal of Investigative Dermatology 135, 1108-1118 (2015).
10. M. Hosseini, K. Ezzedine, A. Taieb, H. R. Rezvani, Oxidative and Energy Metabolism as Potential Clues for Clinical Heterogeneity in Nucleotide Excision Repair Disorders. J Invest Dermatol 135, 341-351 (2015).
11. K. Cao et al., Progerin and telomere dysfunction collaborate to trigger cellular senescence in normal human fibroblasts. J Clin Invest 121, 2833-2844 (2011).
12. P. Scaffidi, T. Misteli, Lamin A-dependent misregulation of adult stem cells associated with accelerated ageing. Nature cell biology 10, 452-459 (2008).
13. J. Espada et al., Nuclear envelope defects cause stem cell dysfunction in premature-aging mice. The Journal of cell biology 181, 27-35 (2008).
14. C. D. Ragnauth et al., Prelamin A Acts to Accelerate Smooth Muscle Cell Senescence and Is a Novel Biomarker of Human Vascular Aging. Circulation 121, 2200-2210 (2010).
15. M. Olive et al., Cardiovascular pathology in Hutchinson-Gilford progeria: correlation with the vascular pathology of aging. Arterioscler Thromb Vasc Biol 30, 2301-2309 (2010).
16. F. J. Blanco, C. Bernabeu, The Splicing Factor SRSF1 as a Marker for Endothelial Senescence. Front Physiol 3, 54 (2012).
17. H.-J. Jung et al., Regulation of prelamin A but not lamin C by miR-9, a brain-specific microRNA. Proceedings of the National Academy of Sciences 109, E423-E431 (2012).
18. X. Nissan et al., Unique Preservation of Neural Cells in Hutchinson- Gilford Progeria Syndrome Is Due to the Expression of the Neural-Specific miR-9 MicroRNA. Cell reports 2, 1-9 (2012).
19. J. C. Kovacic, P. Moreno, V. Hachinski, E. G. Nabel, V. Fuster, Cellular senescence, vascular disease, and aging: Part 1 of a 2-part review. Circulation 123, 1650-1660 (2011).
20. Y. Takamori, T. Wakabayashi, T. Mori, J. Kosaka, H. Yamada, Organization and cellular arrangement of two neurogenic regions in the adult ferret (Mustela putorius furo) brain. The Journal of comparative neurology 522, 1818-1838 (2014).
21. H.-M. Gao, H. Zhou, J.-S. Hong, NADPH oxidases: novel therapeutic targets for neurodegenerative diseases. Trends in Pharmacological Sciences 33, 295-303 (2012).
22. P. B. Campos, B. S. Paulsen, S. K. Rehen, Accelerating neuronal aging in in vitro model brain disorders: a focus on reactive oxygen species. Frontiers in aging neuroscience 6, 292 (2014).
23. A. Barascu et al., Oxydative stress alters nuclear shape through lamins dysregulation: a route to senescence. Nucleus (Austin, Tex.) 3, 411-417 (2012).
24. L. B. Gordon et al., Clinical trial of a farnesyltransferase inhibitor in children with Hutchinson-Gilford progeria syndrome. Proceedings of the National Academy of Sciences 109, 16666-16671 (2012).
25. J. Couzin-Frankel, Medicine. Drug trial offers uncertain start in race to save children with progeria. Science 337, 1594-1595 (2012).
26. S. A. Holstein, R. J. Hohl, Is there a future for prenyltransferase inhibitors in cancer therapy? Current Opinion in Pharmacology 12, 704-709 (2012).
27. Y. Lin, Y. Zheng, Approaches of targeting Rho GTPases in cancer drug discovery. Expert opinion on drug discovery, 1-20 (2015).
28. E. T. Efuet, K. Keyomarsi, Farnesyl and geranylgeranyl transferase inhibitors induce G1 arrest by targeting the proteasome. Cancer research 66, 1040-1051 (2006).
29. S. G. Young, S. H. Yang, B. S. Davies, H. J. Jung, L. G. Fong, Targeting protein prenylation in progeria. Science translational medicine 5, 171ps173 (2013).
30. P. Scaffidi, T. Misteli, Reversal of the cellular phenotype in the premature aging disease Hutchinson-Gilford progeria syndrome. Nat Med 11, 440-445 (2005).
31. F. G. Osorio et al., Splicing-directed therapy in a new mouse model of human accelerated aging. Science translational medicine 3, 106ra107 (2011).
32. I. C. Lopez-Mejia et al., A conserved splicing mechanism of the LMNA gene controls premature aging. Human molecular genetics 20, 4540-4555 (2011).
33. K. Cao et al., Rapamycin Reverses Cellular Phenotypes and Enhances Mutant Protein Clearance in Hutchinson-Gilford Progeria Syndrome Cells. Science translational medicine 3, 89ra58 (2011).
34. V. Cenni et al., Autophagic degradation of farnesylated prelamin A as a therapeutic approach to lamin-linked progeria. Eur J Histochem 55, e36 (2011).
35. Z. Dou et al., Autophagy mediates degradation of nuclear lamina. Nature 527, 105-109 (2015).
36. A. von Mikecz, The nuclear ubiquitin-proteasome system. Journal of cell science 119, 1977-1984 (2006).
37. Q. S. Padiath et al., Lamin B1 duplications cause autosomal dominant leukodystrophy. Nature genetics 38, 1114-1123 (2006).
38. F. G. Osorio et al., Nuclear lamina defects cause ATM-dependent NF-kappaB activation and link accelerated aging to a systemic inflammatory response. Genes & development 26, 2311-2324 (2012).
39. C. A. Ascoli, G. G. Maul, Identification of a novel nuclear domain. The Journal of cell biology 112, 785-795 (1991).
40. S. Das, A. R. Krainer, Emerging Functions of SRSF1, Splicing Factor and Oncoprotein, in RNA Metabolism and Cancer. Mol Cancer Res 12, 1195-1204 (2014).
41. K. Takahashi et al., Induction of pluripotent stem cells from adult human fibroblasts by defined factors. Cell 131, 861-872 (2007).
42. L. Aubry et al., Striatal progenitors derived from human ES cells mature into DARPP32 neurons in vitro and in quinolinic acid-lesioned rats. Proceedings of the National Academy of Sciences of the United States of America 105, 16707-16712 (2008).
43. K. Giraud-Triboult et al., Combined mRNA and microRNA profiling reveals that miR-148a and miR-20b control human mesenchymal stem cell phenotype via EPAS1. Physiological genomics 43, 77-86 (2011).
44. H. Guenou et al., Human embryonic stem-cell derivatives for full reconstruction of the pluristratified epidermis: a preclinical study. Lancet 374, 1745-1753 (2009).
45. X. Nissan et al., Functional melanocytes derived from human pluripotent stem cells engraft into pluristratified epidermis. Proceedings of the National Academy of Sciences of the United States of America 108, 14861-14866 (2011).
46. S. Levenberg, L. S. Ferreira, L. Chen-Konak, T. P. Kraehenbuehl, R. Langer, Isolation, differentiation and characterization of vascular cells derived from human embryonic stem cells. Nature protocols 5, 1115-1126 (2010).
47. T. D. Rockel, D. Stuhlmann, A. von Mikecz, Proteasomes degrade proteins in focal subdomains of the human cell nucleus. Journal of cell science 118, 5231-5242 (2005).
48. G. Ferbeyre, PML a target of translocations in APL is a regulator of cellular senescence. Leukemia 16, 1918-1926 (2002).
49. R. P. Fabunmi, W. C. Wigley, P. J. Thomas, G. N. DeMartino, Interferon gamma regulates accumulation of the proteasome activator PA28 and immunoproteasomes at nuclear PML bodies. Journal of cell science 114, 29-36 (2001).
50. V. Lallemand-Breitenbach et al., Role of promyelocytic leukemia (PML) sumolation in nuclear body formation, 11S proteasome recruitment, and As2O3-induced PML or PML/retinoic acid receptor alpha degradation. The Journal of experimental medicine 193, 1361-1371 (2001).
51. M. Lafarga et al., Clastosome: a subtype of nuclear body enriched in 19S and 20S proteasomes, ubiquitin, and protein substrates of proteasome. Molecular biology of the cell 13, 2771-2782 (2002).
52. V. Lallemand-Breitenbach et al., Arsenic degrades PML or PML-RARalpha through a SUMO-triggered RNF4/ubiquitin-mediated pathway. Nature cell biology 10, 547-555 (2008).
53. S. A. Richards, J. Muter, P. Ritchie, G. Lattanzi, C. J. Hutchison, The accumulation of un-repairable DNA damage in laminopathy progeria fibroblasts is caused by ROS generation and is prevented by treatment with N-acetyl cysteine. Human molecular genetics 20, 3997-4004 (2011).
54. G. Viteri, Y. W. Chung, E. R. Stadtman, Effect of progerin on the accumulation of oxidized proteins in fibroblasts from Hutchinson Gilford progeria patients. Mechanisms of ageing and development 131, 2-8 (2010).
55. J. Adams et al., Potent and selective inhibitors of the proteasome: Dipeptidyl boronic acids. Bioorganic & medicinal chemistry letters 8, 333-338 (1998).
56. S. Meiners et al., Nontoxic proteasome inhibition activates a protective antioxidant defense response in endothelial cells. Free radical biology & medicine 40, 2232-2241 (2006).
57. T. Pan et al., Neuroprotection of rapamycin in lactacystin-induced neurodegeneration via autophagy enhancement. Neurobiology of disease 32, 16-25 (2008).
58. W. X. Ding et al., Linking of autophagy to ubiquitin-proteasome system is important for the regulation of endoplasmic reticulum stress and cell viability. The American journal of pathology 171, 513-524 (2007).
59. V. I. Korolchuk, F. M. Menzies, D. C. Rubinsztein, Mechanisms of cross-talk between the ubiquitin-proteasome and autophagy-lysosome systems. FEBS letters 584, 1393-1398 (2010).
60. X. J. Wang et al., A novel crosstalk between two major protein degradation systems: regulation of proteasomal activity by autophagy. Autophagy 9, 1500-1508 (2013).
61. T. Fernandes-Alnemri, G. Litwack, E. S. Alnemri, Mch2, a new member of the apoptotic Ced-3/Ice cysteine protease gene family. Cancer research 55, 2737-2742 (1995).
62. K. Orth, A. M. Chinnaiyan, M. Garg, C. J. Froelich, V. M. Dixit, The CED-3/ICE-like protease Mch2 is activated during apoptosis and cleaves the death substrate lamin A. The Journal of biological chemistry 271, 16443-16446 (1996).
63. E. A. Slee, C. Adrain, S. J. Martin, Executioner caspase-3, -6, and -7 perform distinct, non-redundant roles during the demolition phase of apoptosis. The Journal of biological chemistry 276, 7320-7326 (2001).
64. A. Takahashi et al., Cleavage of lamin A by Mch2 alpha but not CPP32: multiple interleukin 1 beta-converting enzyme-related proteases with distinct substrate recognition properties are active in apoptosis. Proceedings of the National Academy of Sciences of the United States of America 93, 8395-8400 (1996).
65. M. Fukuda et al., CRM1 is responsible for intracellular transport mediated by the nuclear export signal. Nature 390, 308-311 (1997).
66. W. H. De Vos et al., Repetitive disruptions of the nuclear envelope invoke temporary loss of cellular compartmentalization in laminopathies. Human molecular genetics 20, 4175-4186 (2011).
67. T. Kruger, U. Scheer, p53 localizes to intranucleolar regions distinct from the ribosome production compartments. Journal of cell science 123, 1203-1208 (2010).
68. V. R. Moulton, A. R. Gillooly, G. C. Tsokos, Ubiquitination Regulates Expression of the Serine/Arginine-rich Splicing Factor 1 (SRSF1) in Normal and Systemic Lupus Erythematosus (SLE) T Cells. The Journal of biological chemistry 289, 4126-4134 (2014).
69. X. Nissan et al., Unique preservation of neural cells in Hutchinson- Gilford progeria syndrome is due to the expression of the neural-specific miR-9 microRNA. Cell reports 2, 1-9 (2012).
70. J. Zhang et al., A human iPSC model of Hutchinson Gilford Progeria reveals vascular smooth muscle and mesenchymal stem cell defects. Cell stem cell 8, 31-45 (2011).
71. C. Coffinier et al., Direct synthesis of lamin A, bypassing prelamin a processing, causes misshapen nuclei in fibroblasts but no detectable pathology in mice. The Journal of biological chemistry 285, 20818-20826 (2010).
72. Abu-Baker, G. A. Rouleau, Oculopharyngeal muscular dystrophy: recent advances in the understanding of the molecular pathogenic mechanisms and treatment strategies. Biochim Biophys Acta 1772, 173-185 (2007).
73. J. E. Davies, Z. Berger, D. C. Rubinsztein, Oculopharyngeal muscular dystrophy: potential therapies for an aggregate-associated disorder. The international journal of biochemistry & cell biology 38, 1457-1462 (2006).
74. Brais et al., Short GCG expansions in the PABP2 gene cause oculopharyngeal muscular dystrophy. Nature Genetics 18, 164-167 (1998).
75. Abu-Baker et al., Involvement of the ubiquitin-proteasome pathway and molecular chaperones in oculopharyngeal muscular dystrophy. Human molecular genetics 12, 2609-2623 (2003).
76. Y. P. Bao, S. Sarkar, E. Uyama, D. C. Rubinsztein, Congo red, doxycycline, and HSP70 overexpression reduce aggregate formation and cell death in cell models of oculopharyngeal muscular dystrophy. Journal of medical genetics 41, 47-51 (2004).
77. Calado et al., Nuclear inclusions in oculopharyngeal muscular dystrophy consist of poly(A) binding protein II aggregates which sequester poly(A) RNA. Hum. Mol. Genet. 9, 2321-2328. (2000).
78. J. P. Tavanez et al., Hsp70 chaperones and type I PRMTs are sequestered at intranuclear inclusions caused by polyalanine expansions in PABPN1. PloS one 4, e6418 (2009).
79. L. P. Corbeil-Girard et al., PABPN1 overexpression leads to upregulation of genes encoding nuclear proteins that are sequestered in oculopharyngeal muscular dystrophy nuclear inclusions. Neurobiology of disease 18, 551-567 (2005).
80. X. Fan et al., HnRNP A1 and A/B interaction with PABPN1 in oculopharyngeal muscular dystrophy. Can J Neurol Sci 30, 244-251 (2003).
81. Benoit et al., An essential cytoplasmic function for the nuclear poly(A) binding protein, PABP2, in poly(A) tail length control and early development in Drosophila. Dev Cell 9, 511-522 (2005).
82. B. Benoit et al., The Drosophila poly(A)-binding protein II is ubiquitous throughout Drosophila development and has the same function in mRNA polyadenylation as its bovine homolog in vitro. Nucleic Acids Res. 27, 3771-3778 (1999).
83. Y. Kerwitz et al., Stimulation of poly(A) polymerase through a direct interaction with the nuclear poly(A) binding protein allosterically regulated by RNA. The EMBO journal 22, 3705-3714 (2003).
84. U. Kuhn et al., Poly(A) tail length is controlled by the nuclear poly(A)-binding protein regulating the interaction between poly(A) polymerase and the cleavage and polyadenylation specificity factor. The Journal of biological chemistry 284, 22803-22814 (2009).
85. L. H. Apponi et al., Loss of nuclear poly(A)-binding protein 1 causes defects in myogenesis and mRNA biogenesis. Human molecular genetics 19, 1058-1065 (2010).
86. de Klerk et al., Poly(A) binding protein nuclear 1 levels affect alternative polyadenylation. Nucleic Acids Res 40, 9089-9101 (2012).
87. M. Jenal et al., The poly(A)-binding protein nuclear 1 suppresses alternative cleavage and polyadenylation sites. Cell 149, 538-553 (2012).
88. Y. B. Beaulieu, C. L. Kleinman, A. M. Landry-Voyer, J. Majewski, F. Bachand, Polyadenylation-dependent control of long noncoding RNA expression by the poly(A)-binding protein nuclear 1. PLoS Genet 8, e1003078 (2012).
89. Chartier, B. Benoit, M. Simonelig, A Drosophila model of oculopharyngeal muscular dystrophy reveals intrinsic toxicity of PABPN1. The EMBO journal 25, 2253-2262 (2006).
90. Chartier et al., Mitochondrial dysfunction reveals the role of mRNA poly(A) tail regulation in oculopharyngeal muscular dystrophy pathogenesis. PLoS genetics 11, e1005092 (2015).
91. S. Y. Anvar et al., Deregulation of the ubiquitin-proteasome system is the predominant molecular pathology in OPMD animal models and patients. Skelet Muscle 1, 15 (2011).
92. N. Barbezier et al., Antiprion drugs 6-aminophenanthridine and guanabenz reduce PABPN1 toxicity and aggregation in oculopharyngeal muscular dystrophy. EMBO Mol Med 3, 35-49 (2011).
93. Trollet et al., Molecular and phenotypic characterization of a mouse model of oculopharyngeal muscular dystrophy reveals severe muscular atrophy restricted to fast glycolytic fibres. Human molecular genetics 19, 2191-2207 (2010).

## Claims

1. A modified tripeptide or one of its pharmaceutically acceptable salts, for use in treating and/or preventing a disorder selected from:
- progeroid syndromes;
- oculopharyngeal muscular dystrophy ; and,
- human cancers overexpressing SRSF1 proto-oncogene selected from breast cancer, lung cancer, prostate cancer, kidney cancer, colorectal cancer and skin cancer;
wherein said modified tripeptide is selected from Z-Leu-Leu-Leu-al (MG132), Z-Leu-Leu-nVal-al (MG115), Z-Leu-Leu-Leu-B(OH)₂ (MG262), or Z-Leu-Leu-Phe-al (MG110).

2. The modified tripeptide or one of its pharmaceutically acceptable salts, for use according to claim 1, wherein said disorder is a progeroid syndrome chosen from Hutchinson-Gilford progeria syndrome, atypical progeria syndrome, restrictive dermopathy, Nestor-Guillermo progeria syndrome, Werner syndrome, Bloom syndrome, Rothmund-Thomson syndrome, Cockayne syndrome, *Xeroderma pigmentosum* and trichothiodystrophy.

3. The modified tripeptide or one of its pharmaceutically acceptable salts, for use according to any one of Claims 1 to 2, wherein said modified tripeptide is comprised in a pharmaceutical composition formulated for a topical, oral, parenteral, intranasal, intravenous, intramuscular, subcutaneous or intraocular administration.

4. The modified tripeptide or one of its pharmaceutically acceptable salts, for use according to any one of Claims 1 to 2, wherein said modified tripeptide is used in combination with at least one other drug chosen from anti-inflammatory agents; antisense oligonucleotide SEQ ID NO:1, antisense oligonucleotide of SEQ ID NO:2, aminobisphosphonate inhibitors of farnesyl-pyrophosphate synthase, statin inhibitors of HMG-CoA reductase, NOX1 inhibitors; activators of chaperone or of their ATPase activity; autophagy activators, lithium salts, spermidine, Rho kinase inhibitors, Rho inhibitors and mixtures thereof.

5. The modified tripeptide or one of its pharmaceutically acceptable salts, for use according to any one of Claims 1 to 3, wherein said modified tripeptide or its pharmaceutically acceptable salts is used in combination with at least one other drug chosen from chemotherapy or immunotherapy.

6. A pharmaceutical composition for use in treating and/or preventing a disorder selected from:
- progeroid syndromes; or,
- oculopharyngeal muscular dystrophy; or
- human cancers overexpressing SRSF1 proto-oncogene selected from breast cancer, lung cancer, prostate cancer, kidney cancer, colorectal cancer and skin cancer,
wherein said pharmaceutical composition comprises a modified tripeptide selected from Z-Leu-Leu-Leu-al (MG132), Z-Leu-Leu-nVal-al (MG115), Z-Leu-Leu-Leu-B(OH)₂ (MG262), or Z-Leu-Leu-Phe-al (MG110), or one of their pharmaceutically acceptable salts, combined with pharmaceutically acceptable excipients, and optionally sustained-release matrices.

7. The pharmaceutical composition for use according to claim 6, wherein said disorder is a progeroid syndrome chosen from Hutchinson-Gilford progeria syndrome, atypical progeria syndrome, restrictive dermopathy, Nestor-Guillermo progeria syndrome, Werner syndrome, Bloom syndrome, Rothmund-Thomson syndrome, Cockayne syndrome, *Xeroderma pigmentosum* and trichothiodystrophy.

8. The pharmaceutical composition for use according to any one of Claims 6 to 7, wherein said modified tripeptide is formulated for a topical, oral, parenteral, intranasal, intravenous, intramuscular, subcutaneous or intraocular administration.

9. The pharmaceutical composition for use according to any one of Claims 6 to 8, which is an injectable formulation.

10. A dermatological composition for use in preventing and/or attenuating skin ageing, said composition comprising a modified tripeptide selected from Z-Leu-Leu-Leu-al (MG132), Z-Leu-Leu-nVal-al (MG115), Z-Leu-Leu-Leu-B(OH)₂ (MG262), or Z-Leu-Leu-Phe-al (MG110) or their pharmaceutically acceptable salts, wherein said dermatological composition is formulated for topical administration.

11. A cosmetic use of modified tripeptide or one of its pharmaceutically acceptable salts, for attenuating physiological skin ageing, wherein said modified tripeptide is selected from Z-Leu-Leu-Leu-al (MG132), Z-Leu-Leu-nVal-al (MG115), Z-Leu-Leu-Leu-B(OH)₂ (MG262), or Z-Leu-Leu-Phe-al (MG110)or their pharmaceutically acceptable salts.

12. The cosmetic use of Claim 11, for reducing wrinkles, fine lines, and/or for preventing thinning of the skin and/or an aspect of soft and withered skin.

## Patentansprüche

1. Ein modifiziertes Tripeptid oder eines seiner pharmazeutisch verträglichen Salze zur Verwendung bei der Behandlung und/oder Prävention einer Störung, ausgewählt aus:
- Progeroid-Syndrome;
- oculopharyngeale Muskeldystrophie; und,
- menschliche Krebsarten, die das SRSF1-Protoonkogen überexprimieren und aus Brustkrebs, Lungenkrebs, Prostatakrebs, Nierenkrebs, Darmkrebs und Hautkrebs ausgewählt sind;
wobei das modifizierte Tripeptid ausgewählt ist aus Z-Leu-Leu-Leu-al (MG132), Z-Leu-Leu-nVal-al (MG115), Z-Leu-Leu-Leu-B(OH)₂ (MG262) oder Z-Leu-Leu-Phe-al (MG110).

2. Das modifizierte Tripeptid oder eines seiner pharmazeutisch verträglichen Salze zur Verwendung nach Anspruch 1, wobei die Störung ein Progeroid-Syndrome ist, das ausgewählt aus dem Hutchinson-Gilford-Progeriesyndrom, atypischem Progeriesyndrom, restriktiver Dermopathie, Nestor-Guillermo-Progeriesyndrom, Werner-Syndrom, Bloom-Syndrom, Rothmund-Thomson-Syndrom, Cockayne-Syndrom, *Xeroderma pigmentosum* und Trichothiodystrophie ist.

3. Das modifizierte Tripeptid oder eines seiner pharmazeutisch verträglichen Salze zur Verwendung nach einem der Ansprüche 1 bis 2, wobei das modifizierte Tripeptid von einer pharmazeutischen Zusammensetzung beinhaltet ist, die für eine topische, orale, parenterale, intranasale, intravenöse, intramuskuläre, subkutane oder intraokulare Verabreichung formuliert ist.

4. Das modifizierte Tripeptid oder eines seiner pharmazeutisch verträglichen Salze zur Verwendung nach einem der Ansprüche 1 bis 2, wobei das modifizierte Tripeptid in Kombination mit mindestens einem anderen Medikament verwendet wird, das aus entzündungshemmenden Mitteln t; Antisense-Oligonukleotid SEQ ID NO:1, Antisense-Oligonukleotid von SEQ ID NO:2, Aminobisphosphonat-Inhibitoren der Farnesyl-Pyrophosphat-Synthase, Statin-Inhibitoren der HMG-CoA-Reduktase, NOX1-Inhibitoren; Aktivatoren des Chaperons oder ihrer ATPase-Aktivität; Autophagie-Aktivatoren, Lithiumsalze, Spermidin, Rho-Kinase-Inhibitoren, Rho-Inhibitoren und Mischungen davon ausgewählt ist.

5. Das modifizierte Tripeptid oder eines seiner pharmazeutisch verträglichen Salze zur Verwendung nach einem der Ansprüche 1 bis 3, wobei das modifizierte Tripeptid oder seine pharmazeutisch verträglichen Salze in Kombination mit mindestens einem anderen Medikament, ausgewählt aus Chemotherapie oder Immuntherapie, verwendet wird.

6. Eine pharmazeutische Zusammensetzung zur Verwendung bei der Behandlung und/oder Prävention einer Störung, ausgewählt aus:
- Progeroid-Syndrome; oder,
- oculopharyngeale Muskeldystrophie; oder
- menschliche Krebsarten, die das SRSF1-Protoonkogen überexprimieren und aus Brustkrebs, Lungenkrebs, Prostatakrebs, Nierenkrebs, Darmkrebs und Hautkrebs ausgewählt sind,
wobei die pharmazeutische Zusammensetzung ein modifiziertes Tripeptid , ausgewählt aus Z-Leu-Leu-Leu-al (MG132), Z-Leu-Leu-nVal-al (MG115), Z-Leu-Leu-Leu-B(OH)₂ (MG262) oder Z-Leu-Leu-Phe-al (MG110), oder eines ihrer pharmazeutisch verträglichen Salze, kombiniert mit pharmazeutisch verträglichen Hilfsstoffen, und gegebenenfalls Matrizen zur verzögerten Freisetzung, beinhaltet.

7. Die pharmazeutische Zusammensetzung zur Verwendung nach Anspruch 6, wobei die Störung ein Progeroidsyndrom ist, ausgewählt aus Hutchinson-Gilford Progerie-Syndrom, atypischem Progerie-Syndrom, restriktiver Dermopathie, Nestor-Guillermo Progerie-Syndrom, Werner-Syndrom, Bloom-Syndrom, Rothmund-Thomson-Syndrom, Cockayne-Syndrom, *Xeroderma pigmentosum* und Trichothiodystrophie.

8. Die pharmazeutische Zusammensetzung zur Verwendung nach einem der Ansprüche 6 bis 7, wobei das modifizierte Tripeptid für eine topische, orale, parenterale, intranasale, intravenöse, intramuskuläre, subkutane oder intraokulare Verabreichung formuliert ist.

9. Die pharmazeutische Zusammensetzung zur Verwendung nach einem der Ansprüche 6 bis 8, die eine injizierbare Formulierung ist.

10. Eine dermatologische Zusammensetzung zur Verwendung bei der Vermeidung und/oder Verminderung der Hautalterung, wobei die Zusammensetzung ein modifiziertes Tripeptid beinhaltet, das ausgewählt aus Z-Leu-Leu-Leu-al (MG132), Z-Leu-Leu-nVal-al (MG115), Z-Leu-Leu-Leu-B(OH)₂ (MG262) oder Z-Leu-Leu-Phe-al (MG110) oder deren pharmazeutisch verträglichen Salzen ist, wobei die dermatologische Zusammensetzung für eine topische Verabreichung formuliert ist.

11. Eine kosmetische Verwendung von modifiziertem Tripeptid oder einem seiner pharmazeutisch verträglichen Salze zur Verminderung der physiologischen Hautalterung, wobei das modifizierte Tripeptid ausgewählt ist aus Z-Leu-Leu-Leu-al (MG132), Z-Leu-Leu-nVal-al (MG115), Z-Leu-Leu-Leu-B(OH)₂ (MG262) oder Z-Leu-Leu-Phe-al (MG110) oder deren pharmazeutisch verträglichen Salzen.

12. Die kosmetische Verwendung nach Anspruch 11 zur Reduzierung von Falten, feinen Linien und/oder zur Verhinderung der Verdünnung der Haut und/oder eines Aspekts weicher und verwelkter Haut.

## Revendications

1. Tripeptide modifié ou l'un de ses sels pharmaceutiquement acceptables, pour une utilisation dans le traitement et/ou la prévention d'un trouble sélectionné parmi :
- les syndromes progéroïdes ;
- la dystrophie musculaire oculopharyngée ; et,
- les cancers humains surexprimant un proto-oncogène SRSF1 sélectionné parmi un cancer du sein, un cancer du poumon, un cancer de la prostate, un cancer du rein, un cancer colorectal et un cancer de la peau ;
dans lequel ledit tripeptide modifié est sélectionné parmi Z-Leu-Leu-Leu-al (MG132), Z-Leu-Leu-nVal-al (MG115), Z-Leu-Leu-Leu-B(OH)₂ (MG262), ou Z-Leu-Leu-Phe-al (MG110).

2. Tripeptide modifié ou l'un de ses sels pharmaceutiquement acceptables, pour une utilisation selon la revendication 1, dans lequel ledit trouble est un syndrome progéroïde choisi parmi le syndrome de progeria de Hutchinson-Gilford, le syndrome de progeria atypique, la dermopathie restrictive, le syndrome de progeria de Nestor-Guillermo, le syndrome de Werner, le syndrome de Bloom, le syndrome de Rothmund-Thomson, le syndrome de Cockayne, *Xeroderma pigmentosum* et la trichothiodystrophie.

3. Tripeptide modifié ou l'un de ses sels pharmaceutiquement acceptables, pour une utilisation selon l'une quelconque des revendications 1 à 2, dans lequel ledit tripeptide modifié est compris dans une composition pharmaceutique formulée pour une administration topique, orale, parentérale, intranasale, intraveineuse, intramusculaire, sous-cutanée ou intraoculaire.

4. Tripeptide modifié ou l'un de ses sels pharmaceutiquement acceptables, pour une utilisation selon l'une quelconque des revendications 1 à 2, dans lequel ledit tripeptide modifié est utilisé en combinaison avec au moins un autre médicament choisi parmi les agents anti-inflammatoires ; un oligonucléotide antisens SEQ ID NO : 1, un oligonucléotide antisens de SEQ ID NO : 2, les inhibiteurs aminobisphosphonate de farnésyl-pyrophosphate synthase, les inhibiteurs statine de HMG-CoA réductase, les inhibiteurs de NOX1, les activateurs de chaperone ou de leur activité ATPase ; les activateurs d'autophagie, les sels de lithium, la spermidine, les inhibiteurs de Rho-kinase, les inhibiteurs de Rho et les mélanges de ceux-ci.

5. Tripeptide modifié ou l'un de ses sels pharmaceutiquement acceptables, pour une utilisation selon l'une quelconque des revendications 1 à 3, dans lequel ledit tripeptide modifié ou ses sels pharmaceutiquement acceptables sont utilisés en combinaison avec au moins un autre médicament choisi parmi une chimiothérapie ou une immunothérapie.

6. Composition pharmaceutique pour une utilisation dans le traitement et/ou la prévention d'un trouble sélectionné parmi :
- les syndromes progéroïdes ; ou,
- la dystrophie musculaire oculopharyngée ; ou
- les cancers humains surexprimant un proto-oncogène SRSF1 sélectionné parmi un cancer du sein, un cancer du poumon, un cancer de la prostate, un cancer du rein, un cancer colorectal et un cancer de la peau ;
dans laquelle ladite composition pharmaceutique comprend un tripeptide modifié sélectionné parmi Z-Leu-Leu-Leu-al (MG132), Z-Leu-Leu-nVal-al (MG115), Z-Leu-Leu-Leu-B(OH)₂ (MG262), ou Z-Leu-Leu-Phe-al (MG110), ou l'un de leurs sels pharmaceutiquement acceptables, combiné avec des excipients pharmaceutiquement acceptables, et facultativement des matrices à libération prolongée.

7. Composition pharmaceutique pour une utilisation selon la revendication 6, dans laquelle ledit trouble est un syndrome progéroïde choisi parmi le syndrome de progeria de Hutchinson-Gilford, le syndrome de progeria atypique, la dermopathie restrictive, le syndrome de progeria de Nestor-Guillermo, le syndrome de Werner, le syndrome de Bloom, le syndrome de Rothmund-Thomson, le syndrome de Cockayne, *Xeroderma pigmentosum* et la trichothiodystrophie.

8. Composition pharmaceutique pour une utilisation selon l'une quelconque des revendications 6 à 7, dans laquelle ledit tripeptide modifié est formulé pour une administration topique, orale, parentérale, intranasale, intraveineuse, intramusculaire, sous-cutanée ou intraoculaire.

9. Composition pharmaceutique pour une utilisation selon l'une quelconque des revendications 6 à 8, qui est une formulation injectable.

10. Composition dermatologique pour une utilisation dans la prévention et/ou l'atténuation d'un vieillissement cutané, ladite composition comprenant un tripeptide modifié sélectionné parmi Z-Leu-Leu-Leu-al (MG132), Z-Leu-Leu-nVal-al (MG115), Z-Leu-Leu-Leu-B(OH)₂ (MG262), ou Z-Leu-Leu-Phe-al (MG110), ou leurs sels pharmaceutiquement acceptables, dans laquelle ladite composition dermatologique est formulée pour une administration topique.

11. Utilisation cosmétique d'un tripeptide modifié ou de l'un de ses sels pharmaceutiquement acceptables, pour atténuer un vieillissement cutané physiologique, dans laquelle ledit tripeptide modifié sélectionné parmi Z-Leu-Leu-Leu-al (MG132), Z-Leu-Leu-nVal-al (MG115), Z-Leu-Leu-Leu-B(OH)₂ (MG262), ou Z-Leu-Leu-Phe-al (MG110), ou leurs sels pharmaceutiquement acceptables.

12. Utilisation cosmétique selon la revendication 11, pour réduire les rides, les lignes fines, et/ou pour prévenir un amincissement de la peau et/ou un aspect de peau douce et flétrie.


### SEQUENCE LISTING

<110> A completer
<120> Proteasome inhibitors for treating premature aging disorders
<130> BCT160002QT
<160> 19
<170> PatentIn version 3.5
<210> 1
   <211> 25
   <212> DNA
   <213> Artificial Sequence
<220>
   <223> antisense oligonucleotide
<400> 1
   gctaccactc acgtggtggt gatgg 25
<210> 2
   <211> 25
   <212> DNA
   <213> Artificial Sequence
<220>
   <223> antisense oligonucleotide
<400> 2
   gggtccaccc acctgggctc ctgag 25
<210> 3
   <211> 17
   <212> DNA
   <213> Artificial Sequence
<220>
   <223> primer forward progerin
<400> 3
   actgcagcag ctcgggg 17
<210> 4
   <211> 16
   <212> DNA
   <213> Artificial Sequence
<220>
   <223> primer reverse progerin
<400> 4
   tctgggggct ctgggc 16
<210> 5
   <211> 15
   <212> DNA
   <213> Artificial Sequence
<220>
   <223> probe progerin
<400> 5
   cgctgagtac aacct 15
<210> 6
   <211> 21
   <212> DNA
   <213> Artificial Sequence
<220>
   <223> primer forward lamin A
<400> 6
   tcttctgcct ccagtgtcac g 21
<210> 7
   <211> 19
   <212> DNA
   <213> Artificial Sequence
<220>
   <223> primer reverse lamin A
<400> 7
   agttctgggg gctctgggt 19
<210> 8
   <211> 14
   <212> DNA
   <213> Artificial Sequence
<220>
   <223> probe lamin A
<400> 8
   actcgcagct accg 14
<210> 9
   <211> 22
   <212> DNA
   <213> Artificial Sequence
<220>
   <223> primer forward lamin C
<400> 9
   caactccact ggggaagaag tg 22
<210> 10
   <211> 17
   <212> DNA
   <213> Artificial Sequence
<220>
   <223> primer reverse lamin C
<400> 10
   cggcggctac cactcac 17
<210> 11
   <211> 15
   <212> DNA
   <213> Artificial Sequence
<220>
   <223> probe lamin C
<400> 11
   atgcgcaagc tggtg 15
<210> 12
   <211> 9
   <212> PRT
   <213> Artificial Sequence
<220>
   <223> polyarginine
<400> 12
   Arg Arg Arg Arg Arg Arg Arg Arg Arg
   1               5
<210> 13
   <211> 17
   <212> PRT
   <213> Artificial Sequence
<220>
   <223> antennapedia leader peptide
<400> 13
   Lys Lys Trp Lys Met Arg Arg Asn Gln Phe Trp Val Lys Val Gln Arg
   1               5                   10                  15
   Gly
<210> 14
   <211> 27
   <212> PRT
   <213> Artificial Sequence
<220>
   <223> Bcl-2 binding peptide
<400> 14
   Lys Asn Leu Trp Ala Ala Gln Arg Tyr Gly Arg Glu Leu Arg Arg Met
   1               5                   10                  15
   Ser Asp Glu Phe Glu Gly Ser Phe Lys Gly Leu
               20                  25
<210> 15
   <211> 9
   <212> PRT
   <213> Artificial Sequence
<220>
   <223> tat sequence
<400> 15
   Arg Lys Lys Arg Arg Gln Arg Arg Arg
   1               5
<210> 16
   <211> 21
   <212> PRT
   <213> Artificial Sequence
<220>
   <223> buforin
<400> 16
   Thr Arg Ser Ser Arg Ala Gly Leu Gln Phe Pro Val Gly Arg Val His
   1               5                   10                  15
   Arg Leu Leu Arg Lys
               20
<210> 17
   <211> 13
   <212> PRT
   <213> Artificial Sequence
<220>
   <223> peptidic fragment of the Human T-cell Lymphotrophic Virus (HTLV)-11 Rex
<400> 17
   Thr Arg Arg Gln Arg Thr Arg Arg Ala Arg Arg Asn Arg
   1               5                   10
<210> 18
   <211> 15
   <212> PRT
   <213> Artificial Sequence
<220>
   <223> lipid membrane translocating peptide
<400> 18
   Lys Lys Ala Ala Ala Val Leu Leu Pro Val Leu Leu Ala Ala Pro
   1               5                   10                  15
<210> 19
   <211> 18
   <212> PRT
   <213> Artificial Sequence
<220>
   <223> penetratin
<400> 20